# EUROPEAN PATENT APPLICATION

(11) **EP 2 308 896 A1**
(43) Date of publication of application: **13.04.2011**
(21) Application number: 09290778.1
(22) Date of filing: 09.10.2009
(51) Int. Cl.: C07K 16/28, A61K 38/00

(54) **Polypeptides for binding to the "receptor for advanced glycation endproducts" as well as compositions and methods involving the same**

(71) Applicant: Sanofi-Aventis, 75013 Paris (FR)
(72) Inventor: The designation of the inventor has not yet been filed
(74) Representative: Schneider, Rudolf

(57) **Abstract**

The present invention relates to a polypeptide or polypeptide complex comprising at least the two amino acid sequences arranged to allow for specific binding to the "receptor for advanced glycation endproducts" (RAGE), one or more nucleic acid(s) coding for the polypeptide or polypeptide complex, a cell producing an antibody against RAGE, a pharmaceutical composition comprising at least one polypeptide or nucleic as defined above, optionally for treating a RAGE-related disease or disorder and a method of diagnosing a RAGE-related disease or disorder.

SEQUENCE LISTING

<110>  Sanofi-Aventis

<120>  Polypeptides for binding to the "receptor for advanced glycation
       endproducts" as well as compositions and methods involving the
       same

<130>  DE2009/192

<160>  51

<170>  PatentIn version 3.5

<210>  1
<211>  107
<212>  PRT
<213>  Artificial Sequence

<220>
<223>  variable region for light chain

<400>  1

Asp Ile Val Met Thr Gln Ser Gln Lys Phe Met Ser Thr Ser Val Gly
1               5                   10                  15

Asp Arg Val Ser Val Thr Cys Lys Ala Ser Gln Asn Val Gly Ile Asn
            20                  25                  30

Val Ala Trp Tyr Gln Gln Lys Pro Gly Gln Ser Pro Lys Ala Leu Ile
        35                  40                  45

Tyr Ser Ala Ser Tyr Arg Tyr Ser Gly Val Pro Asp Arg Phe Thr Gly
    50                  55                  60

Ser Gly Ser Gly Thr Asp Phe Thr Leu Ile Ile Ser Asn Val Gln Ser
65                  70                  75                  80

Glu Asp Leu Ala Glu Tyr Phe Cys Gln Gln Tyr Asn Asn Tyr Pro Arg
                85                  90                  95

Thr Phe Gly Gly Gly Thr Lys Leu Glu Ile Lys
            100                 105

<210>  2
<211>  109
<212>  PRT
<213>  Artificial Sequence

<220>
<223>  variable region for light chain

<400>  2

Gln Ala Val Val Thr Gln Glu Ser Ala Leu Thr Thr Ser Pro Gly Glu
1               5                   10                  15

Thr Val Thr Leu Thr Cys Arg Ser Ser Thr Gly Ala Val Thr Thr Ser
            20                  25                  30

Asn Tyr Ala Asn Trp Val Gln Glu Lys Pro Asp His Leu Phe Thr Gly
        35                  40                  45

Leu Thr Gly Gly Thr Asn Asn Arg Ala Pro Gly Val Pro Ala Arg Phe
    50                  55                  60

Ser Gly Ser Leu Ile Gly Asp Lys Ala Ala Leu Thr Ile Thr Gly Ala
65                  70                  75                  80

Gln Thr Glu Asp Glu Ala Ile Tyr Phe Cys Ala Leu Trp Tyr Ser Asn
                85                  90                  95

His Trp Val Phe Gly Gly Gly Thr Lys Leu Thr Val Leu
            100                 105

<210>  3
<211>  106
<212>  PRT
<213>  Artificial Sequence

<220>
<223>  variable region for light chain

<400>  3

Gln Ile Val Leu Thr Gln Ser Pro Ala Ile Met Ser Ala Ser Pro Gly
1               5                   10                  15

Glu Lys Val Thr Met Thr Cys Ser Ala Ser Ser Ser Val Ser Tyr Met
            20                  25                  30

His Trp Tyr Gln Gln Lys Ser Gly Thr Ser Pro Lys Arg Trp Ile Ser
        35                  40                  45

Asp Thr Ser Lys Leu Ala Ser Gly Val Pro Ala Arg Phe Ser Gly Ser
    50                  55                  60

Gly Ser Gly Thr Ser Tyr Ser Leu Thr Ile Ser Ser Met Glu Ala Glu
65                  70                  75                  80

Asp Ala Ala Thr Tyr Tyr Cys Gln Gln Trp Ser Ser Asn Pro Pro Thr
                85                  90                  95

Phe Gly Gly Gly Thr Lys Leu Glu Ile Lys
            100                 105

<210>  4
<211>  108
<212>  PRT
<213>  Artificial Sequence

<220>
<223>  variable region for light chain

<400>  4

Gln Ile Val Leu Thr Gln Ser Pro Ala Ile Met Ser Ala Ser Pro Gly
1               5                   10                  15

Glu Lys Val Thr Ile Ser Cys Ser Ala Ser Ser Ser Val Ser Tyr Met
            20                  25                  30

Tyr Trp Tyr Gln Gln Lys Pro Gly Ser Ser Pro Lys Pro Trp Ile Tyr
        35                  40                  45

Arg Thr Ser Asn Leu Ala Ser Gly Val Pro Ala Arg Phe Ser Gly Ser
    50                  55                  60

Gly Ser Gly Thr Ser Tyr Ser Leu Thr Ile Ser Ser Met Glu Ala Glu
65                  70                  75                  80

Asp Ala Ala Thr Tyr Tyr Cys Gln Gln Tyr His Ser Tyr Pro Pro Met
                85                  90                  95

Tyr Thr Phe Gly Gly Gly Thr Lys Leu Glu Ile Lys
            100                 105

<210>  5
<211>  110
<212>  PRT
<213>  Artificial Sequence

<220>
<223>  variable region for light chain

<400>  5

Asp Ile Val Leu Thr Gln Ser Pro Ala Ser Leu Ala Val Ser Leu Gly
1               5                   10                  15

Gln Arg Ala Thr Ile Ser Cys Arg Ala Ser Lys Ser Val Gly Thr Ser
            20                  25                  30

Asp Ser Ser Tyr Met His Trp Tyr Gln Gln Lys Pro Gly Gln Pro Pro
        35                  40                  45

Lys Leu Leu Ile Tyr Leu Ala Ser Asn Leu Glu Ser Gly Val Pro Ala
    50                  55                  60

Arg Phe Ser Gly Ser Gly Ser Gly Thr Asp Phe Thr Leu Asn Ile His
65                  70                  75                  80

Pro Val Glu Glu Glu Asp Ala Ala Thr Tyr Tyr Cys Gln His Ser Arg
                85                  90                  95

Glu Leu Tyr Thr Phe Gly Gly Gly Thr Lys Leu Glu Ile Lys
            100                 105                 110

<210> 6
<211> 113
<212> PRT
<213> Artificial Sequence

<220>
<223> variable region for light chain

<400> 6

Ser Asp Val Val Leu Thr Gln Thr Pro Leu Ser Leu Pro Val Asn Ile
1               5                   10                  15

Gly Asp Gln Ala Ser Ile Ser Cys Lys Ser Thr Lys Ser Leu Leu Asn
            20                  25                  30

Ser Asp Gly Phe Thr Tyr Leu Asp Trp Tyr Leu Gln Lys Pro Gly Gln
        35                  40                  45

Ser Pro Gln Leu Leu Ile Tyr Leu Val Ser Asn Arg Phe Ser Gly Val
    50                  55                  60

Pro Asp Arg Phe Ser Gly Ser Gly Ser Gly Thr Asp Phe Thr Leu Lys
65                  70                  75                  80

Ile Ser Arg Val Glu Ala Glu Asp Leu Gly Val Tyr Tyr Cys Phe Gln
                85                  90                  95

Ser Asn Tyr Leu Pro Leu Thr Phe Gly Gly Gly Thr Lys Val Glu Ile
            100                 105                 110

Lys

<210> 7
<211> 107
<212> PRT
<213> Artificial Sequence

<220>
<223> variable region for light chain

<400> 7

Asn Ile Val Met Thr Gln Ser Pro Lys Ser Met Ser Met Ser Val Gly
1               5                   10                  15

Glu Arg Val Thr Leu Ser Cys Lys Ala Ser Glu Asn Val Gly Thr Tyr
            20                  25                  30

Val Ser Trp Tyr Gln Gln Lys Pro Glu Gln Ser Pro Lys Leu Leu Ile
        35                  40                  45

Tyr Gly Ala Ser Asn Arg Tyr Thr Gly Val Pro Asp Arg Phe Thr Gly
    50                  55                  60

Ser Gly Ser Ala Thr Asp Phe Thr Leu Thr Ile Ser Ser Val Gln Ala
65                  70                  75                  80

Glu Asp Leu Ala Asp Tyr His Cys Gly Gln Ser Tyr Thr Tyr Pro Tyr
                85                  90                  95

Thr Phe Gly Gly Gly Thr Lys Leu Glu Ile Lys
            100                 105

<210>  8
<211>  112
<212>  PRT
<213>  Artificial Sequence

<220>
<223>  variable region for light chain

<400>  8

Asn Ile Met Met Thr Gln Ser Pro Ser Ser Leu Ala Val Ser Ala Gly
1               5                   10                  15

Glu Lys Val Thr Met Ser Cys Lys Ser Ser Gln Ser Val Leu Tyr Ser
            20                  25                  30

Ser Asn Gln Lys Asn Tyr Leu Ala Trp Tyr Gln Gln Lys Pro Gly Gln
        35                  40                  45

Ser Pro Lys Leu Leu Ile Tyr Trp Ala Ser Thr Arg Glu Ser Gly Val
    50                  55                  60

Pro Asp Arg Phe Thr Gly Ser Gly Ser Gly Thr Asp Phe Thr Leu Thr
65                  70                  75                  80

Ile Ser Ser Val Gln Ala Glu Asp Leu Ala Val Tyr Tyr Cys His Gln
                85                  90                  95

Tyr Leu Ser Ser Tyr Thr Phe Gly Gly Gly Thr Lys Leu Glu Ile Lys
            100                 105                 110

<210>  9
<211>  107
<212>  PRT
<213>  Artificial Sequence

<220>
<223>  variable region for light chain

<400>  9

Asp Ile Val Met Thr Gln Ser Gln Lys Phe Met Ser Thr Ser Val Gly
1               5                   10                  15

Asp Arg Val Ser Val Thr Cys Lys Ala Ser Gln Asn Val Gly Thr Asn
            20                  25                  30

Val Ala Trp Tyr Gln Gln Lys Pro Gly Gln Ser Pro Lys Ala Leu Ile
        35                  40                  45

Tyr Ser Ala Ser Tyr Arg Tyr Ser Gly Val Pro Asp Arg Phe Thr Gly
    50                  55                  60

Ser Gly Ser Gly Thr Asp Phe Thr Leu Thr Ile Ser Asn Val Gln Ser
65                  70                  75                  80

Glu Asp Leu Ala Glu Tyr Phe Cys Gln Gln Tyr Asn Ser Tyr Pro Leu
                85                  90                  95

Thr Phe Gly Ala Gly Thr Lys Leu Glu Leu Lys
            100                 105

<210>  10
<211>  107
<212>  PRT
<213>  Artificial Sequence

<220>
<223>  variable region for light chain

<400>  10

Asn Ile Val Met Thr Gln Ser Pro Lys Ser Met Ser Met Ser Val Gly
1               5                   10                  15

Glu Arg Val Thr Leu Ser Cys Lys Ala Ser Glu Asn Val Gly Thr Tyr
            20                  25                  30

Val Ser Trp Tyr Gln Gln Lys Pro Glu Gln Ser Pro Lys Leu Leu Ile
        35                  40                  45

Tyr Gly Ala Ser Asn Arg Tyr Thr Gly Val Pro Asp Arg Phe Thr Gly
    50                  55                  60

Ser Gly Ser Ala Thr Asp Phe Thr Leu Thr Ile Ser Ser Val Gln Ala
65                  70                  75                  80

Glu Asp Leu Ala Asp Tyr His Cys Gly Gln Ser Tyr Ser Tyr Pro Tyr
                85                  90                  95

Thr Phe Gly Gly Gly Thr Lys Leu Glu Ile Lys
            100                 105

<210>  11
<211>  111
<212>  PRT
<213>  Artificial Sequence

<220>
<223>  variable region for light chain

<400>  11

Asp Ile Val Leu Thr Gln Ser Pro Ala Ser Leu Ala Val Ser Leu Gly
1               5                   10                  15

Gln Arg Ala Thr Ile Ser Cys Arg Ala Ser Lys Ser Val Ser Thr Ser
            20                  25                  30

Gly Tyr Ser Tyr Met His Trp Tyr Gln Gln Lys Pro Gly Gln Pro Pro
        35                  40                  45

Lys Leu Leu Ile Tyr Leu Ala Ser His Leu Glu Ser Gly Val Pro Ala
    50                  55                  60

Arg Phe Ser Gly Ser Gly Ser Gly Thr Asp Phe Ser Leu Asn Ile His
65                  70                  75                  80

Pro Val Glu Glu Glu Asp Ala Ala Thr Tyr Tyr Cys Gln His Ser Arg
                85                  90                  95

Glu Leu Pro Trp Thr Phe Gly Gly Gly Thr Lys Leu Glu Ile Lys
            100                 105                 110

<210>  12
<211>  110
<212>  PRT
<213>  Artificial Sequence

<220>
<223>  variable region for light chain

<400>  12

Gln Ala Val Val Thr Gln Glu Ser Ala Leu Thr Thr Ser Pro Gly Glu
1               5                   10                  15

Thr Val Thr Leu Thr Cys Arg Ser Ser Thr Gly Ala Val Thr Thr Ser
            20                  25                  30

Asn Tyr Ala Asn Trp Val Gln Glu Lys Pro Asp His Leu Phe Thr Gly
        35                  40                  45

Leu Ile Gly Gly Thr Asn Asn Arg Ala Pro Gly Val Pro Ala Arg Phe
    50                  55                  60

Ser Gly Ser Leu Ile Gly Asp Lys Ala Ala Leu Thr Ile Thr Gly Ala
65                  70                  75                  80

Gln Thr Glu Asp Glu Ala Ile Tyr Phe Cys Ala Leu Trp Tyr Ser Asn
                85                  90                  95

His Phe Trp Val Phe Gly Gly Gly Thr Lys Leu Thr Val Leu
            100                 105                 110

<210>  13
<211>  113

<212>  PRT
<213>  Artificial Sequence

<220>
<223>  variable region for light chain

<400>  13

Ser Asp Val Val Leu Thr Gln Thr Pro Leu Ser Leu Pro Val Ser Ile
1               5                   10                  15

Gly Asp Gln Ala Ser Ile Ser Cys Lys Ser Thr Lys Ser Leu Leu Asn
            20                  25                  30

Ser Asp Gly Phe Thr Tyr Leu Asp Trp Tyr Leu Gln Lys Pro Gly Gln
        35                  40                  45

Ser Pro Gln Leu Leu Ile Tyr Leu Val Ser Asn Arg Phe Ser Gly Val
    50                  55                  60

Pro Asp Ser Phe Ser Gly Ser Gly Ser Gly Thr Asp Phe Thr Leu Lys
65                  70                  75                  80

Ile Ser Arg Val Glu Ala Glu Asp Leu Gly Val Tyr Tyr Cys Phe Gln
                85                  90                  95

Ser Asn Tyr Phe Pro Leu Thr Phe Gly Gly Gly Thr Thr Leu Glu Ile
            100                 105                 110

Lys

<210>  14
<211>  107
<212>  PRT
<213>  Artificial Sequence

<220>
<223>  variable region for light chain

<400>  14

Asp Ile Val Met Thr Gln Ser Gln Lys Phe Met Ser Thr Ser Val Gly
1               5                   10                  15

Asp Arg Val Ser Val Thr Cys Lys Ala Ser Gln Asn Val Gly Thr Asn
            20                  25                  30

Val Ala Trp Tyr Gln Gln Lys Pro Gly Gln Ser Pro Lys Ala Leu Ile
        35                  40                  45

Tyr Ser Ala Ser Tyr Arg Tyr Ser Gly Val Pro Asp Arg Phe Thr Gly
    50                  55                  60

Ser Gly Ser Gly Thr Asp Phe Thr Leu Thr Ile Ser Asn Val Gln Ser
65                  70                  75                  80

Glu Asp Leu Ala Glu Phe Phe Cys Gln Gln Tyr Asn Ser Tyr Pro Leu
                85                  90                  95

Thr Phe Gly Ala Gly Thr Lys Leu Glu Leu Lys
            100                 105

<210>  15
<211>  113
<212>  PRT
<213>  Artificial Sequence

<220>
<223>  variable region for light chain

<400>  15

Asp Ile Val Met Ser Gln Ser Pro Ser Ser Leu Ala Val Ser Val Gly
1               5                   10                  15

Glu Lys Val Thr Met Ser Cys Lys Ser Ser Gln Thr Leu Leu Tyr Ser
            20                  25                  30

Ser Asn Gln Lys Asn Tyr Leu Ala Trp Tyr Gln Gln Lys Pro Gly Gln
        35                  40                  45

Ser Leu Lys Leu Leu Ile Tyr Trp Ala Ser Thr Arg Glu Ser Gly Val
    50                  55                  60

Pro Asp Arg Phe Ala Gly Ser Gly Ser Gly Thr Asp Phe Thr Leu Thr
65                  70                  75                  80

Ile Ser Ser Val Lys Ala Glu Asp Leu Ala Val Tyr Tyr Cys Gln Gln
                85                  90                  95

Tyr Phe Gly Tyr Pro Tyr Thr Phe Gly Gly Gly Thr Lys Leu Glu Ile
            100                 105                 110

Lys

<210>  16
<211>  109
<212>  PRT
<213>  Artificial Sequence

<220>
<223>  variable region for light chain

<400>  16

Gln Ala Val Val Thr Gln Glu Ser Ala Leu Thr Thr Ser Pro Gly Glu
1               5                   10                  15

Thr Val Thr Leu Thr Cys Arg Ser Ser Thr Gly Ala Val Thr Thr Ser
            20                  25                  30

Asn Tyr Ala Asn Trp Val Gln Glu Lys Pro Asp His Leu Phe Thr Gly
        35                  40                  45

Leu Ile Gly Gly Thr Asn Asn Arg Ala Pro Gly Val Pro Ala Arg Phe
    50                  55                  60

Ser Gly Ser Leu Ile Gly Asp Lys Ala Ala Leu Thr Ile Thr Gly Ala
65                  70                  75                  80

Gln Thr Glu Asp Glu Ala Ile Tyr Phe Cys Ala Leu Trp Tyr Ser Asn
                85                  90                  95

His Trp Val Phe Gly Gly Gly Thr Lys Leu Thr Val Leu
            100                 105

<210>  17
<211>  109
<212>  PRT
<213>  Artificial Sequence

<220>
<223>  variable region for light chain

<400>  17

Gln Ala Val Val Thr Gln Glu Ser Ala Leu Thr Thr Ser Pro Gly Glu
1               5                   10                  15

Thr Val Thr Leu Thr Cys Arg Ser Ser Thr Gly Ala Val Thr Thr Ser
            20                  25                  30

Asn Tyr Ala Asn Trp Val Gln Glu Lys Pro Asp His Leu Phe Thr Gly
        35                  40                  45

Leu Ile Arg Gly Thr Asn Asn Arg Ala Pro Gly Val Pro Ala Arg Phe
    50                  55                  60

Ser Gly Ser Leu Ile Gly Asp Lys Ala Ala Leu Thr Ile Thr Gly Ala
65                  70                  75                  80

Gln Thr Glu Asp Glu Ala Ile Tyr Phe Cys Ala Leu Trp Tyr Ser Asn
                85                  90                  95

His Trp Val Phe Gly Gly Gly Thr Lys Leu Thr Val Leu
            100                 105

<210>  18
<211>  111
<212>  PRT
<213>  Artificial Sequence

<220>
<223>  variable region for light chain

<400> 18

Asp Ile Val Leu Thr Gln Ser Pro Ala Ser Leu Ala Val Ser Leu Gly
1               5                   10                  15

Gln Arg Ala Thr Ile Ser Cys Arg Ala Ser Lys Ser Val Ser Ile Ser
            20                  25                  30

Gly Tyr Ser Tyr Leu His Trp Asn Gln Gln Lys Pro Gly Gln Ser Pro
        35                  40                  45

Lys Leu Leu Ile Tyr Leu Ala Ser Asn Leu Glu Ser Gly Val Pro Ala
    50                  55                  60

Arg Phe Ser Gly Ser Gly Ser Gly Thr Asp Phe Thr Leu Asn Ile His
65                  70                  75                  80

Pro Val Glu Glu Glu Asp Ala Ala Thr Tyr Tyr Cys Gln His Ser Arg
                85                  90                  95

Glu Leu Pro Tyr Thr Phe Gly Gly Gly Thr Lys Leu Glu Ile Lys
            100                 105                 110

<210>  19
<211>  108
<212>  PRT
<213>  Artificial Sequence

<220>
<223>  variable region for light chain

<400>  19

Asp Ile Val Met Thr Gln Ser Gln Lys Phe Met Ser Thr Ser Val Gly
1               5                   10                  15

Asp Arg Val Ser Ile Thr Cys Lys Ala Ser Gln Asn Val Gly Thr Ala
            20                  25                  30

Val Ala Trp Tyr Gln Gln Lys Pro Gly Gln Ser Pro Lys Leu Leu Ile
        35                  40                  45

Tyr Ser Ala Ser Asn Arg Tyr Thr Gly Val Pro Asp Arg Phe Thr Gly
    50                  55                  60

Ser Gly Ser Gly Thr Asp Phe Thr Leu Thr Ile Ser Asn Met Gln Ser
65                  70                  75                  80

Glu Asp Leu Ala Asp Tyr Phe Cys Gln Gln Tyr Ser Ser Tyr Pro Leu
                85                  90                  95

Leu Thr Phe Gly Ala Gly Thr Lys Leu Glu Leu Lys
            100                 105

<210>  20
<211>  107
<212>  PRT
<213>  Artificial Sequence

<220>
<223>  variable region for light chain

<400>  20

Asp Ile Gln Met Thr Gln Ser Ser Ser Tyr Leu Ser Val Ser Leu Gly
1               5                   10                  15

Gly Arg Val Thr Ile Thr Cys Lys Ala Ser Asp Arg Ile Asn Tyr Trp
            20                  25                  30

Leu Ala Trp Tyr Gln Gln Lys Pro Gly Asn Ala Pro Arg Leu Leu Ile
        35                  40                  45

Ser Gly Ala Thr Thr Leu Glu Thr Gly Val Pro Ser Arg Phe Ser Gly
    50                  55                  60

Ser Gly Ser Gly Lys Asp Tyr Thr Leu Ser Ile Thr Ser Leu Gln Thr
65                  70                  75                  80

Glu Asp Val Ala Thr Tyr Tyr Cys Gln Gln Tyr Trp Ser Thr Pro Tyr
                85                  90                  95

Thr Phe Gly Gly Gly Thr Lys Leu Glu Leu Lys
            100                 105


<210>  21
<211>  107
<212>  PRT
<213>  Artificial Sequence

<220>
<223>  variable region for light chain

<400>  21

Asp Ile Val Met Thr Gln Ser His Lys Phe Met Ser Thr Ser Val Gly
1               5                   10                  15

Asp Arg Val Ser Ile Thr Cys Lys Ala Ser Gln Asp Val Ser Thr Ala
            20                  25                  30

Val Ala Trp Tyr Gln Gln Lys Pro Gly Gln Ser Pro Lys Leu Leu Ile
        35                  40                  45

Tyr Ser Ala Ser Tyr Arg Tyr Thr Gly Val Pro Asp Arg Phe Thr Gly
    50                  55                  60

Ser Gly Ser Gly Thr Asp Phe Thr Phe Thr Ile Ser Ser Val Gln Ala
65                  70                  75                  80

Glu Asp Leu Ala Val Tyr Tyr Cys Gln Gln His Tyr Asn Thr Pro Arg
                85                  90                  95

Thr Phe Gly Gly Gly Thr Lys Leu Glu Ile Lys
            100                 105

<210>  22
<211>  109
<212>  PRT
<213>  Artificial Sequence

<220>
<223>  variable region for light chain

<400>  22

Gln Ala Val Val Thr Gln Glu Ser Ala Leu Thr Thr Ser Pro Gly Glu
1               5                   10                  15

Thr Val Thr Leu Thr Cys Arg Ser Ser Thr Gly Ala Val Thr Thr Ser
            20                  25                  30

Asn Tyr Ala Asn Trp Val Gln Glu Lys Pro Asp His Leu Phe Thr Gly
        35                  40                  45

Leu Ile Gly Gly Thr Asn Asn Arg Ala Pro Asp Val Pro Ala Arg Phe
    50                  55                  60

Ser Gly Ser Leu Ile Gly Asp Lys Ala Ala Leu Thr Ile Thr Gly Ala
65                  70                  75                  80

Gln Thr Glu Asp Glu Ala Ile Tyr Phe Cys Ala Leu Trp Tyr Ser Asn
                85                  90                  95

His Trp Val Phe Gly Gly Gly Thr Lys Leu Thr Val Leu
            100                 105

<210>  23
<211>  109
<212>  PRT
<213>  Artificial Sequence

<220>
<223>  variable region for light chain

<400>  23

Gln Ala Val Val Thr Gln Glu Ser Ala Leu Thr Thr Ser Pro Gly Glu
1               5                   10                  15

Thr Val Thr Leu Thr Cys Arg Ser Ser Thr Gly Ala Val Thr Thr Ser
            20                  25                  30

Asn Tyr Ala Asn Trp Val Gln Glu Lys Pro Asp His Leu Phe Thr Gly
        35                  40                  45

Leu Ile Gly Gly Thr Asn Asn Arg Ser Pro Gly Val Pro Ala Arg Phe
    50                  55                  60

Ser Gly Ser Leu Ile Gly Asp Lys Ala Ala Leu Thr Ile Thr Gly Ala
65                  70                  75                  80

Gln Thr Glu Asp Glu Ala Ile Tyr Phe Cys Ala Leu Trp Tyr Ser Asn
                85                  90                  95

His Leu Val Phe Gly Gly Gly Thr Lys Leu Thr Val Leu
            100                 105

<210>  24
<211>  115
<212>  PRT
<213>  Artificial Sequence

<220>
<223>  variable region for heavy chain

<400>  24

Gln Val Gln Leu Gln Gln Ser Gly Pro Glu Leu Val Lys Pro Gly Ala
1               5                   10                  15

Ser Val Arg Ile Ser Cys Lys Ala Ser Gly Tyr Thr Phe Thr Ser Tyr
            20                  25                  30

Phe Ile His Trp Val Lys Gln Arg Pro Gly Gln Gly Leu Glu Trp Ile
        35                  40                  45

Gly Trp Ile Tyr Pro Gly Asn Val Asn Thr Lys Tyr Asn Glu Lys Phe
    50                  55                  60

Lys Asp Lys Ala Thr Leu Thr Ala Asp Lys Ser Ser Ser Thr Ala Tyr
65                  70                  75                  80

Met Gln Leu Ser Asn Leu Thr Ser Glu Asp Ser Ala Val Tyr Phe Cys
                85                  90                  95

Val Arg Gly Gln Leu Gly Asp Tyr Trp Gly Gln Gly Ile Thr Leu Thr
            100                 105                 110

Val Ser Ser
        115

<210>  25
<211>  117
<212>  PRT
<213>  Artificial Sequence

<220>
<223>  variable region for heavy chain

<400>  25

Gln Val Gln Leu Gln Gln Pro Gly Ala Glu Leu Val Lys Pro Gly Ala
1               5                   10                  15

Ser Val Lys Leu Ser Cys Lys Ala Ser Gly Tyr Thr Phe Thr Ser Tyr
            20                  25                  30

Trp Met His Trp Val Lys Gln Arg Pro Gly Gln Gly Leu Glu Trp Ile
        35                  40                  45

Gly Glu Ser Asn Pro Ser Asn Gly Arg Thr Asn Tyr Asn Glu Lys Phe
    50                  55                  60

Lys Asn Lys Ala Thr Leu Thr Val Asp Lys Ser Ser Ser Thr Ala Tyr
65                  70                  75                  80

Met Gln Leu Ser Ser Leu Thr Ser Glu Asp Ser Ala Val Tyr Tyr Cys
                85                  90                  95

Ala Arg Ala Pro Tyr Tyr Gly Phe Asp Tyr Trp Gly Gln Gly Thr Thr
            100                 105                 110

Leu Thr Val Ser Ser
        115

<210>  26
<211>  119
<212>  PRT
<213>  Artificial Sequence

<220>
<223>  variable region for heavy chain

<400>  26

Glu Val Gln Leu Val Glu Ser Gly Gly Gly Leu Val Lys Pro Gly Gly
1               5                   10                  15

Ser Leu Lys Leu Ser Cys Ala Ala Ser Gly Phe Thr Phe Ser Ser Tyr
            20                  25                  30

Val Met Ser Trp Val Arg Gln Ser Pro Glu Lys Arg Leu Glu Trp Val
        35                  40                  45

Ala Glu Ile Ser Ser Gly Gly Ser Tyr Thr Tyr Tyr Pro Asp Thr Val
    50                  55                  60

Thr Gly Arg Phe Thr Ile Ser Arg Asp Asn Asp Lys Asn Thr Leu Tyr
65                  70                  75                  80

Leu Glu Met Ser Ser Leu Arg Ser Glu Asp Thr Ala Met Tyr Tyr Cys
                85                  90                  95

Ala Arg Pro Pro Tyr Gly Lys Asp Ala Met Asp Tyr Trp Gly Gln Gly
            100                 105                 110

Thr Ser Val Thr Val Ser Ser
        115

<210>  27
<211>  121
<212>  PRT
<213>  Artificial Sequence

<220>
<223>  variable region for heavy chain

<400>  27

Gln Val Gln Leu Gln Gln Pro Gly Ala Glu Leu Val Lys Pro Gly Ala
1               5                   10                  15

Ser Val Arg Leu Ser Cys Lys Ala Ser Gly Tyr Thr Phe Thr Ser Tyr
            20                  25                  30

Trp Met His Trp Val Lys Gln Arg Pro Gly Gln Gly Leu Glu Trp Ile
        35                  40                  45

Gly Glu Ile Asn Pro Ser Asn Gly Arg Thr Asn Tyr Asn Glu Lys Phe
    50                  55                  60

Lys Ser Lys Ala Thr Leu Thr Val Asp Lys Ser Ser Ser Thr Ala Tyr
65                  70                  75                  80

Met Gln Leu Ser Ser Leu Thr Ser Glu Asp Ser Ala Val Tyr Tyr Cys
                85                  90                  95

Ala Arg Asp Gly Leu Gly Tyr Arg Pro Ile Ala Met Asp Tyr Trp Gly
            100                 105                 110

Gln Gly Thr Ser Val Thr Val Ser Ser
        115                 120

<210>  28
<211>  115
<212>  PRT
<213>  Artificial Sequence

<220>
<223>  variable region for heavy chain

<400>  28

Asp Val Gln Leu Gln Glu Ser Gly Pro Asp Leu Val Lys Pro Ser Gln
1               5                   10                  15

Ser Leu Ser Leu Thr Cys Thr Val Thr Gly Tyr Ser Ile Thr Ser Gly
            20                  25                  30

Tyr Ser Trp His Trp Ile Arg Gln Phe Pro Gly Asn Lys Leu Glu Trp
        35                  40                  45

Met Gly Tyr Ile His Tyr Ser Gly Ser Thr Asn Tyr Asn Pro Ser Leu
50                  55                  60

Lys Ser Arg Ile Ser Ile Thr Arg Asp Thr Ser Lys Asn Gln Phe Phe
65                  70                  75                  80

Leu Gln Leu Asn Ser Val Thr Thr Glu Asp Thr Ala Thr Tyr Tyr Cys
                85                  90                  95

Ala Arg Gly Gly Asp Phe Ala Tyr Trp Gly Gln Gly Thr Leu Val Thr
            100                 105                 110

Val Ser Ala
        115

<210>  29
<211>  119
<212>  PRT
<213>  Artificial Sequence

<220>
<223>  variable region for heavy chain

<400>  29

Gln Ile Gln Leu Val Gln Ser Gly Pro Glu Leu Lys Lys Pro Gly Glu
1               5                   10                  15

Thr Val Lys Ile Ser Cys Lys Ala Ser Gly Tyr Thr Phe Thr Asp Tyr
            20                  25                  30

Ser Met His Trp Val Lys Gln Ala Pro Gly Lys Gly Leu Lys Trp Met
        35                  40                  45

Gly Trp Ile Asn Thr Glu Thr Gly Glu Pro Thr Tyr Ala Asp Asp Phe
    50                  55                  60

Lys Gly Arg Phe Ala Phe Ser Leu Glu Thr Ser Ala Ser Thr Ala Tyr
65                  70                  75                  80

Leu Leu Ile Asn Asn Leu Lys Thr Glu Asp Thr Ala Thr Tyr Phe Cys
                85                  90                  95

Ala Arg Asp Tyr Leu Tyr Tyr Tyr Ala Met Asp Tyr Trp Gly Gln Gly
            100                 105                 110

Thr Ser Val Thr Val Ser Ser
        115

<210>  30
<211>  116
<212>  PRT
<213>  Artificial Sequence

<220>
<223>  variable region for heavy chain

<400>  30

Gln Val Gln Leu Gln Gln Pro Gly Ser Glu Leu Val Arg Pro Gly Ala
1               5                   10                  15

Ser Val Lys Leu Ser Cys Lys Ala Ser Gly Tyr Thr Phe Thr Asn Tyr
            20                  25                  30

Trp Met His Trp Val Lys Gln Arg Pro Gly Gln Gly Leu Glu Trp Ile
        35                  40                  45

Gly Asn Ile Tyr Pro Gly Ser Gly Ser Thr Asn Tyr Asp Glu Lys Phe
    50                  55                  60

Lys Ser Lys Ala Thr Leu Thr Val Asp Thr Ser Ser Ser Thr Ala Tyr
65                  70                  75                  80

Met Gln Leu Ser Ser Leu Thr Ser Glu Asp Ser Ala Val Tyr Tyr Cys
                85                  90                  95

Thr Arg Leu Arg Arg Gly Ile Ala Tyr Trp Gly Gln Gly Thr Leu Val
            100                 105                 110

Thr Val Ser Ala
        115


<210>  31
<211>  115
<212>  PRT
<213>  Artificial Sequence

<220>
<223>  variable region for heavy chain

<400>  31

Gln Val Gln Leu Gln Gln Ser Gly Pro Glu Leu Val Lys Pro Gly Thr
1               5                   10                  15

Ser Val Arg Ile Ser Cys Lys Ala Ser Gly Tyr Thr Phe Thr Ser Tyr
            20                  25                  30

Tyr Ile His Trp Val Lys Gln Arg Pro Gly Gln Gly Leu Glu Trp Ile
        35                  40                  45

Gly Trp Ile Tyr Pro Gly Asn Val Ile Thr Asn Tyr His Glu Lys Phe
    50                  55                  60

Lys Gly Lys Ala Ser Leu Thr Ala Asp Lys Ser Ser Ser Thr Ala Tyr
65                  70                  75                  80

Met Gln Leu Ser Ser Leu Thr Ser Glu Asp Ser Ala Val Tyr Phe Cys
                85                  90                  95

Ala Arg Glu Asp Pro Phe Ala Tyr Trp Gly Gln Gly Thr Leu Val Thr
            100                 105                 110

Val Ser Ala
        115

<210>  32
<211>  120
<212>  PRT
<213>  Artificial Sequence

<220>
<223>  variable region for heavy chain

<400>  32

Glu Val Lys Leu Glu Glu Ser Gly Gly Gly Leu Val Gln Pro Gly Gly
1               5                   10                  15

Ser Met Lys Leu Ser Cys Val Ala Ser Gly Phe Thr Phe Ser Asn Tyr
            20                  25                  30

Trp Met Asn Trp Val Arg Gln Ser Pro Glu Lys Gly Leu Glu Trp Val
        35                  40                  45

Ala Glu Ile Arg Leu Lys Ser Asn Asn Tyr Ala Thr His Tyr Ala Glu
    50                  55                  60

Ser Val Lys Gly Arg Phe Thr Ile Ser Arg Asp Asp Ser Lys Ser Ser
65                  70                  75                  80

Val Tyr Leu Gln Met Asn Asp Leu Arg Ala Glu Asp Pro Gly Ile Tyr
                85                  90                  95

Tyr Cys Ile Arg Asp Tyr Gly Asn Tyr Ala Met Asp His Trp Gly Gln
            100                 105                 110

Gly Thr Ser Val Thr Val Ser Ser
        115                 120

<210>  33
<211>  116
<212>  PRT
<213>  Artificial Sequence

<220>
<223>  variable region for heavy chain

<400>  33

Glu Val Gln Leu Gln Gln Ser Gly Thr Val Leu Ala Arg Pro Gly Ala
1               5                   10                  15

Ser Val Lys Met Ser Cys Lys Ala Ser Gly Tyr Ser Phe Thr Ser Tyr
            20                  25                  30

Trp Met His Trp Val Lys Gln Arg Pro Gly Gln Gly Leu Glu Trp Ile
        35                  40                  45

Gly Ala Ile Phe Pro Gly Asn Ser Asp Thr Thr Tyr Asn Gln Lys Phe
    50                  55                  60

Lys Gly Lys Ala Lys Leu Thr Ala Val Thr Ser Ala Ser Thr Ala Tyr
65                  70                  75                  80

Met Glu Leu Ser Ser Leu Thr Asn Glu Asp Ser Ala Val Tyr Tyr Cys
                85                  90                  95

Thr Gly Leu Arg Arg Gly Phe Pro Tyr Trp Gly Gln Gly Thr Leu Val
            100                 105                 110

Thr Val Ser Val
        115

<210> 34
<211> 120
<212> PRT
<213> Artificial Sequence

<220>
<223> variable region for heavy chain

<400> 34

Gln Val Gln Leu Gln Gln Ser Gly Ala Glu Leu Val Arg Pro Gly Thr
1               5                   10                  15

Ser Val Lys Val Ser Cys Lys Ala Ser Gly Tyr Ala Phe Thr Asn Tyr
            20                  25                  30

Leu Ile Glu Trp Val Lys Gln Arg Pro Gly Gln Gly Leu Glu Trp Ile
        35                  40                  45

Gly Met Ile Asn Pro Gly Ser Gly Gly Thr Asn Tyr Asn Glu Lys Phe
    50                  55                  60

Lys Gly Lys Ala Thr Leu Thr Ala Asp Lys Ser Ser Ser Thr Ala Tyr
65                  70                  75                  80

Met Gln Leu Ser Ser Leu Thr Ser Asp Asp Ser Ala Val Tyr Phe Cys
                85                  90                  95

Ala Arg Gly Arg Gly Gly His Tyr Arg Tyr Phe Asp Val Trp Gly Ala
            100                 105                 110

Gly Thr Thr Val Thr Val Ser Ser
        115                 120

<210> 35
<211> 119
<212> PRT
<213> Artificial Sequence

<220>
<223> variable region for heavy chain

<400> 35

His Ser Glu Ile Gln Leu Gln Gln Thr Gly Pro Glu Leu Val Lys Pro
1               5                   10                  15

Gly Ala Ser Val Lys Ile Ser Cys Lys Ala Ser Gly Tyr Ser Phe Thr
            20                  25                  30

Asp Tyr Ile Met Val Trp Val Lys Gln Ser His Gly Lys Ser Leu Glu
        35                  40                  45

Trp Ile Gly Thr Ile Asn Pro Tyr Tyr Gly Ser Thr Ser Tyr Asn Leu
    50                  55                  60

Lys Phe Lys Gly Lys Ala Thr Leu Thr Val Asp Lys Ser Ser Ser Thr
65                  70                  75                  80

Ala Asn Met Gln Leu Asn Ser Leu Thr Ser Glu Asp Ser Ala Val Tyr
                85                  90                  95

Tyr Cys Ala Arg Leu Arg Leu Tyr Ala Met Asp Tyr Trp Gly Gln Gly
            100                 105                 110

Thr Ser Val Thr Val Ser Ser
        115

<210> 36
<211> 119
<212> PRT
<213> Artificial Sequence

<220>
<223> variable region for heavy chain

<400> 36

Gln Ile Gln Leu Val Gln Ser Gly Pro Glu Leu Lys Lys Pro Gly Glu
1               5                   10                  15

Thr Val Lys Ile Ser Cys Lys Ala Ser Gly Tyr Thr Phe Thr Asp Tyr
            20                  25                  30

Ser Met His Trp Val Lys Gln Ala Pro Gly Lys Gly Leu Lys Trp Met
        35                  40                  45

Gly Trp Ile Asn Thr Glu Thr Gly Glu Pro Thr Tyr Ala Asp Asp Phe
    50                  55                  60

Lys Gly Arg Phe Ala Phe Ser Leu Glu Thr Ser Ala Ser Thr Ala Tyr
65                  70                  75                  80

Leu Gln Ile Asn Asn Leu Lys Asn Glu Asp Thr Ala Thr Tyr Phe Cys
                85                  90                  95

Ala Arg Asp Tyr Leu Tyr Tyr Tyr Ala Met Asp Tyr Trp Gly Gln Gly
            100                 105                 110

Thr Ser Val Thr Val Ser Ser
        115

<210>  37
<211>  120
<212>  PRT
<213>  Artificial Sequence

<220>
<223>  variable region for heavy chain

<400>  37

Glu Val Lys Leu Glu Glu Ser Gly Gly Gly Leu Val Gln Pro Gly Gly
1               5                   10                  15

Ser Met Lys Leu Ser Cys Val Ala Ser Gly Phe Thr Phe Ser Asn Tyr
            20                  25                  30

Trp Met Asn Trp Val Arg Gln Ser Pro Glu Lys Gly Leu Glu Trp Val
        35                  40                  45

Ala Glu Ile Arg Leu Lys Ser Asn Asn Tyr Ala Thr His Tyr Ala Glu
    50                  55                  60

Ser Val Lys Gly Arg Phe Thr Ile Ser Arg Asp Asp Ser Lys Ser Ser
65                  70                  75                  80

Val Tyr Leu Gln Met Asn Asn Leu Arg Ala Glu Asp Thr Gly Ile Tyr
                85                  90                  95

Phe Cys Ile Arg Asp Tyr Gly Asn Tyr Ala Met Asp Tyr Trp Gly Gln
            100                 105                 110

Gly Thr Ser Val Thr Val Ser Ser
        115                 120

<210>  38
<211>  118
<212>  PRT
<213>  Artificial Sequence

<220>
<223>  variable region for heavy chain

<400>  38

Gln Val Gln Leu Gln Gln Ser Gly Pro Glu Leu Val Lys Pro Gly Ala
1               5                   10                  15

Ser Val Arg Ile Ser Cys Lys Ala Ser Gly Tyr Thr Phe Thr Asp Tyr
            20                  25                  30

Tyr Ile His Trp Val Lys Gln Arg Pro Gly Gln Gly Leu Glu Trp Ile
        35                  40                  45

Gly Trp Ile Tyr Pro Gly Asn Val Ile Thr Lys Tyr Asn Glu Lys Phe
    50                  55                  60

Lys Gly Lys Ala Thr Leu Thr Ala Asp Lys Ser Ser Ser Thr Ala Tyr
65                  70                  75                  80

Met Gln Leu Ser Ser Leu Thr Ser Glu Asp Ser Ala Val Tyr Phe Cys
                85                  90                  95

Ala Arg Tyr Asp Tyr Asp Tyr Ala Met Asp Tyr Trp Gly Gln Gly Thr
            100                 105                 110

Ser Val Thr Val Ser Ser
        115


<210>  39
<211>  117
<212>  PRT
<213>  Artificial Sequence

<220>
<223>  variable region for heavy chain

<400>  39

Gln Val Gln Leu Gln Gln Ser Gly Ala Glu Leu Val Arg Pro Gly Thr
1               5                   10                  15

Ser Val Lys Val Ser Cys Lys Ala Ser Gly Tyr Ala Phe Thr Asn Tyr
            20                  25                  30

Leu Ile Asp Trp Val Asn Gln Arg Pro Gly Gln Gly Leu Glu Trp Ile
        35                  40                  45

Gly Val Ile Asn Pro Gly Ser Gly Gly Thr Asn Tyr Asn Glu Lys Phe
    50                  55                  60

Thr Gly Lys Ala Thr Leu Thr Ala Asp Lys Ser Ser Ser Thr Ala Tyr
65                  70                  75                  80

Met Gln Leu Ser Ser Leu Thr Ser Asp Asp Ser Ala Val Tyr Phe Cys
                85                  90                  95

Ala Arg Arg Arg Val Asp Thr Met Asp Tyr Trp Gly Gln Gly Thr Ser
            100                 105                 110

Val Thr Val Ser Ser
        115

<210> 40
<211> 118
<212> PRT
<213> Artificial Sequence

<220>
<223> variable region for heavy chain

<400> 40

Gln Val Gln Leu Gln Gln Ser Gly Ala Glu Leu Val Arg Pro Gly Thr
1               5                   10                  15

Ser Val Lys Val Ser Cys Lys Ala Ser Gly Tyr Ala Phe Thr Asn Tyr
            20                  25                  30

Leu Ile Glu Trp Val Lys Gln Arg Pro Gly Gln Gly Leu Glu Trp Ile
        35                  40                  45

Gly Val Ile Asn Pro Gly Ser Gly Gly Thr Asn Tyr Ser Glu Arg Phe
    50                  55                  60

Lys Gly Lys Ala Thr Leu Thr Ala Asp Lys Ser Ser Ser Thr Ala Tyr
65                  70                  75                  80

Met Gln Leu Ser Ser Leu Thr Ser Asp Asp Ser Ala Val Tyr Phe Cys
                85                  90                  95

Ala Ser Tyr Arg Tyr Asp Gly Gly Met Asp Tyr Trp Gly Gln Gly Thr
            100                 105                 110

Ser Val Thr Val Ser Ser
        115

<210> 41
<211> 118
<212> PRT
<213> Artificial Sequence

<220>
<223> variable region for heavy chain

<400> 41

Gln Val Gln Leu Gln Gln Ser Gly Pro Glu Leu Val Arg Pro Gly Ala
1               5                   10                  15

Ser Val Lys Met Ser Cys Lys Ala Ser Gly Tyr Thr Phe Thr Ser Tyr
            20                  25                  30

Trp Met His Trp Val Lys Gln Arg Pro Gly Gln Gly Leu Glu Trp Ile
        35                  40                  45

Gly Met Ile Asp Pro Ser Asn Ser Glu Thr Arg Leu Asn Gln Lys Phe
    50                  55                  60

Lys Asp Lys Ala Thr Leu Asn Val Asp Lys Ser Ser Asn Thr Ala Tyr
65                  70                  75                  80

Met Gln Leu Ser Ser Leu Thr Ser Glu Asp Ser Ala Val Tyr Tyr Cys
                85                  90                  95

Ala Arg Asn Phe Tyr Gly Ser Ser Leu Arg Val Trp Gly Ala Gly Thr
            100                 105                 110

Thr Val Thr Val Ser Ser
        115

<210>  42
<211>  119
<212>  PRT
<213>  Artificial Sequence

<220>
<223>  variable region for heavy chain

<400>  42

Glu Val Lys Leu Val Glu Ser Gly Gly Gly Leu Val Lys Pro Gly Gly
1               5                   10                  15

Ser Leu Lys Leu Ser Cys Ala Ala Ser Gly Phe Ala Phe Ser Ser Tyr
            20                  25                  30

Asp Met Ser Trp Val Arg Gln Thr Pro Glu Lys Arg Leu Glu Trp Val
        35                  40                  45

Ala Thr Ile Ser Ser Gly Gly Ser Tyr Thr Ser Tyr Pro Asp Ser Val
    50                  55                  60

Gln Gly Arg Phe Thr Ile Ser Arg Asp Asn Ala Arg Asn Thr Leu Tyr
65                  70                  75                  80

Leu Gln Met Ser Ser Leu Arg Ser Glu Asp Thr Ala Leu Tyr Tyr Cys
                85                  90                  95

Ala Ser Ser Gln Leu Pro Pro Tyr Ala Met Asp Tyr Trp Gly Gln Gly
            100                 105                 110

Thr Ser Val Thr Val Ser Ser
        115

<210>  43
<211>  118

<212>  PRT
<213>  Artificial Sequence

<220>
<223>  variable region for heavy chain

<400>  43

Gln Val Gln Leu Gln Gln Ser Gly Ala Glu Leu Ala Lys Pro Gly Ala
1               5                   10                  15

Ser Val Lys Met Ser Cys Arg Ala Ser Gly Tyr Thr Phe Thr Asp Tyr
            20                  25                  30

Trp Met His Trp Val Lys Gln Arg Pro Gly Gln Gly Leu Glu Trp Ile
        35                  40                  45

Gly Phe Ile Asn Pro Ser Thr Val Tyr Thr Glu Tyr Ile Pro Lys Phe
    50                  55                  60

Lys Asp Lys Ala Thr Leu Thr Ala Asp Lys Ser Ser Ser Thr Ala Tyr
65                  70                  75                  80

Met Gln Leu Ser Ser Leu Thr Ser Glu Asp Ser Ala Val Tyr Tyr Cys
                85                  90                  95

Ala Arg Ser Asp Gly Gly Trp Tyr Phe Asp Val Trp Gly Ala Gly Thr
            100                 105                 110

Thr Val Thr Val Ser Ser
        115

<210>  44
<211>  113
<212>  PRT
<213>  Artificial Sequence

<220>
<223>  variable region for heavy chain

<400>  44

Glu Val Gln Leu Gln Gln Ser Gly Thr Val Leu Ala Arg Pro Gly Ala
1               5                   10                  15

Ser Val Lys Met Ser Cys Lys Ala Ser Gly Tyr Thr Phe Thr Ser Tyr
            20                  25                  30

Trp Met His Trp Val Lys Gln Arg Pro Gly Gln Gly Leu Glu Trp Ile
        35                  40                  45

Gly Ala Ile Tyr Pro Gly Asp Ser Asp Thr Tyr Tyr Asn Gln Lys Phe
    50                  55                  60

Lys Gly Lys Ala Lys Leu Thr Ala Val Thr Ser Thr Ser Thr Ala Tyr
65                  70                  75                  80

Met Glu Leu Ser Ser Leu Thr Asn Glu Asp Ser Ala Val Tyr Tyr Cys
                85                  90                  95

Thr Arg Asn Trp Asp Tyr Trp Gly Gln Gly Thr Thr Leu Thr Val Ser
            100                 105                 110

Ser

<210>  45
<211>  120
<212>  PRT
<213>  Artificial Sequence

<220>
<223>  variable region for heavy chain

<400>  45

Glu Val Met Leu Val Asp Ser Gly Gly Gly Leu Val Lys Pro Gly Gly
1               5                   10                  15

Ser Leu Lys Leu Ser Cys Ala Ala Ser Gly Phe Thr Phe Arg Ser Tyr
            20                  25                  30

Ala Met Ser Trp Val Arg Gln Thr Pro Glu Lys Arg Leu Glu Trp Val
        35                  40                  45

Ala Thr Ile Ser Ser Gly Gly Ser Tyr Thr Tyr Tyr Pro Asp Ser Val
    50                  55                  60

Arg Gly Arg Phe Thr Thr Ser Arg Asp Asn Gly Lys Asn Thr Leu Tyr
65                  70                  75                  80

Leu Gln Met Ser Ser Leu Arg Ser Glu Asp Thr Ala Met Tyr Tyr Cys
                85                  90                  95

Ala Arg His Gly Gly Asn Tyr Ser Ala Trp Phe Thr Tyr Trp Gly Gln
            100                 105                 110

Gly Thr Leu Val Thr Val Ser Ala
        115                 120

<210>  46
<211>  119
<212>  PRT
<213>  Artificial Sequence

<220>
<223>  variable region for heavy chain

<400>  46

His Ser Glu Ile Gln Leu Gln Gln Thr Gly Pro Glu Leu Val Lys Pro
1               5                   10                  15

Gly Ala Ser Val Lys Ile Ser Cys Lys Ala Ser Gly Tyr Ser Phe Thr
            20                  25                  30

Asp Tyr Ile Met Leu Trp Val Lys Gln Ser His Gly Lys Ser Leu Glu
        35                  40                  45

Trp Ile Gly Asn Ile Asn Pro Tyr Tyr Gly Ser Thr Phe Tyr Asn Leu
    50                  55                  60

Lys Phe Lys Gly Lys Ala Thr Leu Thr Val Asp Lys Ser Ser Ser Thr
65                  70                  75                  80

Ala Tyr Met Gln Leu Asn Ser Leu Thr Ser Glu Asp Ser Ala Val Tyr
                85                  90                  95

Tyr Cys Ala Arg Ser Asp Tyr Trp Tyr Phe Asp Val Trp Gly Ala Gly
            100                 105                 110

Thr Thr Val Thr Val Ser Ser
        115

<210>  47
<211>  4
<212>  PRT
<213>  Artificial Sequence

<220>
<223>  motif after CDR-L3

<220>
<221>  Variant
<222>  (3)..(3)
<223>  Xaa = any amino acid

<400>  47

Phe Gly Xaa Gly
1

<210>  48
<211>  4
<212>  PRT
<213>  Artificial Sequence

<220>
<223>  motif before CDR-H1

<220>
<221>  Variant
<222>  (2)..(2)
<223>  Xaa = any amino acid

<220>
<221>  Variant
<222>  (3)..(3)
<223>  Xaa = any amino acid

<220>
<221>  Variant
<222>  (4)..(4)
<223>  Xaa = any amino acid

<400>  48

Cys Xaa Xaa Xaa
1


<210>  49
<211>  5
<212>  PRT
<213>  Artificial Sequence

<220>
<223>  motif before CDR-H2

<400>  49

Leu Glu Trp Ile Gly
1               5


<210>  50
<211>  4
<212>  PRT
<213>  Artificial Sequence

<220>
<223>  motif after CDR-H3


<220>
<221>  Variant
<222>  (3)..(3)
<223>  Xaa = any amino acid

<400>  50

Trp Gly Xaa Gly
1


<210>  51
<211>  11
<212>  PRT
<213>  Artificial Sequence

<220>
<223>  immunostimulatory substance

<400>  51

Lys Leu Lys Leu Leu Leu Leu Leu Lys Leu Lys
1               5                   10

## Description

The present invention relates to a polypeptide or polypeptide complex comprising at least the two amino acid sequences arranged to allow for specific binding to the "receptor for advanced glycation endproducts" (RAGE), one or more nucleic acid(s) coding for the polypeptide or polypeptide complex, a cell producing an antibody against RAGE, a pharmaceutical composition comprising at least one polypeptide or nucleic as defined above, optionally for treating a RAGE-related disease or disorder and a method of diagnosing a RAGE-related disease or disorder.

The receptor for advanced glycation endproducts (RAGE) is a 35kD transmembrane receptor of the immunoglobulin super family which was first characterized in 1992 by Neeper et al. (Neeper et al., 1992, J.Biol.Chem. 267: 14998-15004). It is a multi-ligand cell surface member of the immunoglobulin super-family. RAGE consists of an extracellular domain, a single membrane-spanning domain, and a cytosolic tail. The extracellular domain of the receptor consists of one V-type immunoglobulin domain followed by two C-type immunoglobulin domains. The cytosolic domain is responsible for signal transduction and the transmembrane domain anchors the receptor in the cell membrane. The variable domain binds the RAGE ligands. RAGE also exists in a soluble form (RAGE).

RAGE's name comes from its ability to bind advanced glycation endproducts (AGE), a heterogeneous group of non-enzymatically altered proteins, which form in prolonged hyperglycemic states. However, AGE's may be only incidental, pathogenic ligands. Besides AGEs, RAGE is also able to bind other ligands and is thus often referred to as a pattern recognition receptor. However, RAGE is an unusual pattern-recognition receptor that binds several different classes of endogenous molecules leading to various cellular responses, including cytokine secretion, increased cellular oxidant stress, neurite outgrowth and cell migration. Known ligands of RAGE include proteins having β-sheet fibrils that are characteristic of amyloid deposits and pro-inflammatory mediators, including S100/calgranulins, serum amyloid (SAA) (fibrillar form), β-amyloid protein (Aβ), and high mobility group box-1 chromosomal protein 1 (HMGB1, also known as amphotehn). HMGB-1 has been shown to be a late mediator of lethality in two models of murine sepsis, and interaction between RAGE and ligands such as HMGB1 is believed to play an important role in the pathogenesis of sepsis and other inflammatory diseases.

RAGE is expressed by many cell types, e.g., endothelial and smooth muscle cells, macrophages and lymphocytes, in many different tissues, including lung, heart, kidney, skeletal muscle and brain. Expression is increased in chronic inflammatory states such as rheumatoid arthritis and diabetic nephropathy.

A number of significant human disorders are associated with an increased production of ligands for RAGE or with increased production of RAGE itself. Due to an enhanced level of RAGE ligands in diabetes or other chronic disorders, this receptor is hypothesised to have a causative effect in a range of inflammatory diseases such as diabetic complications, Alzheimer's disease and even some tumors.

Additionally, RAGE has been linked to several chronic diseases, which are thought to result from vascular damage. The pathogenesis is hypothesized to include ligand binding upon which RAGE signals activation of the nuclear factor kappa B (NF-κB). NF-κB controls several genes which are involved in inflammation. Interestingly, RAGE itself will also be up-regulated by NF-κB. Given a condition, where there is a large amount of RAGE ligands (e.g. AGE in diabetes or Amyloid-β-protein in Alzheimer's disease) this establishes a positive feed-back cycle, which leads to chronic inflammation. This chronic condition is then believed to alter the micro- and macrovasculature in a fatal way which ends in organ damage or even organ failure. Diseases that have been linked to RAGE are many chronic inflammatory diseases, including rheumatoid and psoriatic arthritis and intestinal bowel disease, cancers, diabetes and diabetic nephropathy, amyloidoses, cardiovascular diseases, sepsis, atherosclerosis, peripheral vascular disease, myocardial infarction, congestive heart failure, diabetic retinopathy, diabetic neuropathy, diabetic nephropathy and Alzheimer's disease.

Consistently effective therapeutics are not available for many of these disorders. It would be beneficial to have safe and effective treatments for such RAGE-related disorders. One approach includes the use of polypeptides, e.g. antibodies, binding to RAGE.

Surprisingly, a number of monoclonal antibodies (mABs) has now been identified, which provide for the advantageous characteristics. Particularly, anti-RAGE monoclonal antibodies have been identified based on a set of experimental data including binding constants, cross-reactivity, domain mapping and in vitro functional data (competition ELISA). Based on the above data 23 mAbs have been selected fulfilling the following criteria:
Binding constants KD ≤ 1.0 x 10-9 M and koff ≤ 2.0 x 10-3 s-1

As known to the skilled person, binding characteristics of antibodies are mediated by the variable domains. For binding to an antigen, it is essential that a suitable variable domain from the heavy chain and a co-acting variable domain from the light chain are present and arranged in order to allow for the co-acting. The variable domain is also referred to as the FV region and is the most important region for binding to antigens. More specifically variable loops, three each on the light (VL) and heavy (VH) chains are responsible for binding to the antigen. These loops are referred to as the Complementarity Determining Regions (CDRs). The three loops are referred to as L1, L2 and L3 for VL and H1, H2 and H3 for VH. However, a variety of different arrangements of variable domain from the heavy chain and a co-acting variable domain from the light chain are known in the art. Therefore, the identification of a suitable variable domain from the heavy chain and a co-acting variable domain from the light chain is essential for the present invention. Therefore, their sequences have been identified for the 23 antibodies specified above.

Accordingly, in a first aspect the present invention relates to a polypeptide or polypeptide complex comprising at least the two amino acid sequences or functionally active variants thereof, wherein the least the two amino acid sequences are
- SEQ ID NO: 1 and SEQ ID NO:24,
- SEQ ID NO:2 and SEQ ID NO:25,
- SEQ ID NO:3 and SEQ ID NO:26,
- SEQ ID NO:4 and SEQ ID NO:27,
- SEQ ID NO:5 and SEQ ID NO:28,
- *SEQ ID NO:6* and SEQ ID NO:29,
- SEQ ID NO:7 and SEQ ID NO:30,
- SEQ ID NO:8 and SEQ ID NO:31,
- SEQ ID NO:9 and SEQ ID NO:32,
- SEQ ID NO:10 and SEQ ID NO:33,
- SEQ ID NO:11 and SEQ ID NO:34,
- SEQ ID NO:12 and SEQ ID NO:35,
- SEQ ID NO:13 and SEQ ID NO:36,
- SEQ ID NO:14 and SEQ ID NO:37,
- SEQ ID NO:15 and SEQ ID NO:38,
- SEQ ID NO:16 and SEQ ID NO:39,
- SEQ ID NO: 17 and SEQ ID NO:40,
- SEQ ID NO:18 and SEQ ID NO:41,
- SEQ ID NO: 19 and SEQ ID NO:42,
- SEQ ID NO:20 and SEQ ID NO:43,
- SEQ ID NO:21 and SEQ ID NO:44,
- SEQ ID NO:22 and SEQ ID NO:45, and/or
- SEQ ID NO:23 and SEQ ID NO:46,
wherein these sequences are arranged to allow for specific binding to the "receptor for advanced glycation endproducts" (RAGE).

In accordance with the present invention the polypeptide or polypeptide complex comprises at least the two amino acid sequences or functionally active variants thereof as defined above. The sequences of SEQ ID NO: 1 to 23 are the variable domains of light chains and that of SEQ ID NO: 24 to 46 are the variable domains of heavy chains of the antibodies identified (as determined by sequence analysis). The SEQ ID NO of a variable domain of a heavy chain corresponding to the prevailing variable domain of a light chain may be determined by adding 23 to the SEQ ID NO of said variable domain of a light chain. For example, the SEQ ID NO of the variable domain of a heavy chain corresponding to the variable domain of a light chain of SEQ ID NO: 5 is SEQ ID NO: 28 (5 + 23).

It is essential for the present invention that the polypeptide or polypeptide complex comprises the two co-acting amino acid sequences or functionally active variants thereof, as defined above. If they are arranged in a suitable way, the arrangement allows for specific binding to RAGE. A variety of different antibody formats have been developed or identified so far. Any of these or any other suitable arrangement may be used for the polypeptide or polypeptide complex of the present invention, as long as the format or arrangement allows for specific binding to RAGE.

The two sequences, as defined by the above SEQ ID NOs or variants thereof, may be arranged in one polypeptide or in a peptide complex. If they are arranged in one polypeptide the two sequences may be connected by a linker sequence, preferably a peptide linker, e.g. as a fusion protein. If they are arranged in a polypeptide complex, two or more polypeptides are bound to each other by non-covalent bonding including hydrogen bonds, ionic bonds, Van der Walls forces, and hydrophobic interactions. The above sequences or functionally active variants thereof may constitute the polypeptide or polypeptide complex or may be part thereof.

A polypeptide (also known as proteins) is an organic compound made of α-amino acids arranged in a linear chain. The amino acids in a polymer chain are joined together by the peptide bonds between the carboxyl and amino groups of adjacent amino acid residues. In general, the genetic code specifies 20 standard amino acids. After or even during synthesis, the residues in a protein may be chemically modified by post-translational modification, which alter the physical and chemical properties, folding, stability, activity, and ultimately, the function of the proteins.

Polypeptides or complexes thereof as defined herein selectively recognize and specifically bind to RAGE. Use of the terms "selective" or "specific" herein refers to the fact that the disclosed polypeptides or complexes thereof do not show significant binding to other than RAGE, except in those specific instances where the polypeptide/complexe is supplemented to confer an additional, distinct specificity to the RAGE-specific binding portion (as, for example, in bispecific or bifunctional molecules where the molecule is designed to bind or effect two functions, at least one of which is to specifically bind RAGE). In specific embodiments, RAGE-specific polypeptides or complexes thereof bind to human RAGE with a KD of 1.2 x 10-6 or less. In specific embodiments, RAGE-specific polypeptides or complexes thereof bind to human RAGE with a KD of 5 x 10-7 or less, of 2 x 10-7 or less, or of 1 x 10-7 or less. In additional embodiments, RAGE-specific polypeptides or complexes thereof bind to human RAGE with a ID of 1 x 10-8 or less. In other embodiments, RAGE-specific polypeptides or complexes thereof bind to human RAGE with a KD of 5 x 10-9 or less, or of 1 x 10-9 or less. In further embodiments, RAGE-specific polypeptides or complexes thereof bind to human RAGE with a KD of 1 x 10-10 or less, a KD of 1 x 10-11 or less, or a KD of 1 x 10-12 or less. In specific embodiments, RAGE-specific polypeptides or complexes thereof do not bind other proteins at the above KDs.

KD refers to the dissociation constant obtained from the ratio ofkd (the dissociation rate of a particular binding molecule-target protein interaction; also referred to as koff) to ka (the association rate of the particular binding molecule-target protein interaction; also referred to as kon), or kd/ka which is expressed as a molar concentration (M). KD values can be determined using methods well established in the art. A preferred method for determining the KD of a binding molecule is by using surface plasmon resonance, for example a biosensor system such as a Biacore(TM) (GE Healthcare Life Sciences) system (see Example 5 and Table 2). Another method is shown in Fig. 2 and Example 2.

RAGE-specific polypeptides or complexes thereof have been shown to dose-dependently inhibit RAGE/ligand interaction (see Fig. 4, Example 3 and 4 and Table 1). Accordingly, RAGE-specific polypeptides or complexes thereof may be characterized by their ability to counteract ligand binding to RAGE. The extent of inhibition by any RAGE-specific polypeptide or complex thereof may be measured quantitatively in statistical comparison to a control, or via any alternative method available in the art. In specific embodiments, the inhibition is at least about 10 % inhibition. In other embodiments, the inhibition is at least 20 %, 30 %, 40 %, 50 %, 60 %, 70 %, 80 %, 90 %, or 95 %.

The polypeptide or complex thereof may comprise also a functionally active variant of the above sequences. A functionally active variant of the invention is characterized by having a biological activity similar to that displayed by the complete protein, including the ability to bind to RAGE, and optionally to inhibit RAGE. The variant is functionally active in the context of the present invention, if the activity (e.g. binding activity, optionally expressed as KD) of the variant amounts to at least 10 %, preferably at least 25 %, more preferably at least 50 %, even more preferably at least 70 %, still more preferably at least 80 %, especially at least 90 %, particularly at least 95 %, most preferably at least 99 % of the activity of the peptide/complex without sequence alteration. Suitable methods for determining binding activity to RAGE are given in the Examples. A functionally active variant may be obtained by a limited number of amino acid substitutions, deletions and/or insertions.

In a preferred embodiment of the present invention the functionally active variant of any of the sequences SEQ ID NO: 1 to 23 comprises the complementarity determining region L3 (CDR L3), preferably CDR L1, CDR L2 and CDR L3, of the respective sequence of SEQ ID NO: 1 to 23; and/or the functionally active variant of any of the sequences SEQ ID NO: 24 to 46 comprises the complementarity determining region H3 (CDR H3), preferably CDR H1, CDR H2 and CDR H3, of the respective sequence of SEQ ID NO: 24 to 46. In a most preferred embodiment the functionally active variant of any of the sequences SEQ ID NO: 1 to 23 comprises CDR L1, CDR L2 and CDR L3 of the respective sequence of SEQ ID NO: 1 to 23; and the functionally active variant of any of the sequences SEQ ID NO: 24 to 46 comprises CDR H1, CDR H2 and CDR H3 of the respective sequence of SEQ ID NO: 24 to 46. Alternatively, one of the sequences may be SEQ ID NO: 1 to 46 without any sequence alterations and the other may be a variant as defined herein.

Different methods of identifying CDRs in a sequence of a variable region have been described. Additionally, a series of software programs are known, which may be used for this purpose. However, the following set of rules has been applied to the sequences of SEQ IOD NO: 1 to 46 to identify the CDRs in these sequences (see also www.bioinf.org.uk; MacCallum et al., 1996, J. Mol. Biol. 262 (5): 732-745; Antibody Engineering Lab Manual, Chapter "Protein Sequence and Structure Analysis of Antibody Variable Domains", Ed.: Duebel, S. and Kontermann, R., Springer-Verlag, Heidelberg). The sequences with CDRs indicated are shown in Figure 1,

| CDR-L1 | |
|---|---|
| Start | Approx residue 24 |
| Residue before | always a Cys |
| Residue after | always a Trp. Typically Trp-Tyr-Gln, but also, Trp-Leu-Gln, Trp-Phe-Gln, Trp-Tyr-Leu |
| Length | 10 to 17 residues |

| CDR-L2 | |
|---|---|
| Start | always 16 residues after the end of L1 |
| Residues before | generally Ile-Tyr, but also, Val-Tyr, Ile-Lys, Ile-Phe |
| Length | always 7 residues |

| CDR-L3 | |
|---|---|
| Start | always 33 residues after end of L2 |
| Residue before | always Cys |
| Residues after | always Phe-Gly-XXX-Gly (SEQ ID NO: 47) |
| Length | 7 to 11 residues |

| CDR-H1 | |
|---|---|
| Start | Approx residue 26 (always 4 after a Cys) |
| Residues before | always Cys-XXX-XXX-XXX (SEQ ID NO: 48) |
| Residues after | always a Trp. Typically Trp-Val, but also, Trp-Ile, Trp-Ala |
| Length | 10 to 12 residues |

| CDR-H2 | |
|---|---|
| Start | always 15 residues after the end of CDR-H1 |
| Residues before | typically Leu-Glu-Trp-Ile-Gly (SEQ ID NO: 49) but a number of variations |
| Residues after | Lys/Arg-Leu/Ile/VaL/Phe/Thr/Ala-Thr/Ser/Ile/Ala |
| Length | 9 to 12 residues |

| CDR-H3 | |
|---|---|
| Start | always 33 residues after end of CDR-H2 (always 2 after a Cys) |
| Residues before | always Cys-XXX-XXX (typically Cys-Ala-Arg) |
| Residues after | always Trp-Gly-XXX-Gly (SEQ ID NO: 50) |
| Length | 3 to 25 residues |

As detailed above, within VH and VL there are hypervariable regions which show the most sequence variability from one antibody to another and framework regions which are less variable. Folding brings the hypervariable regions together to form the antigen-binding pockets. These sites of closest contact between antibody and antigen are the CDR of the antibody which mediates the specificity of the antibody. Accordingly, they are of particular importance for antigen binding. Though it is preferred that the functionally active variant comprises all three CDR, it has been found that for some antibodies CDR-L3 and CDR-H3 are sufficient to confer specificity. Accordingly, in one embodiment only the presence of CDR-L3 and CDR-H3 is mandatory. In any case, the CDRs have to be arranged to allow for specific binding to the antigen, here RAGE.

In a preferred embodiment of the present invention the CDRs (CDR-L3 and -H3; or CDR-L1, - L2, -L3, -H1, -H2 and -H3) are arranged in the framework of the prevailing variable domain, i.e. L1, L2 and L3 in the framework of VL and H1, H2 and H3 in the framework of VH. This means that the CDRs as identified by any suitable method or as shown in Fig. 1 may be removed from the shown neighborhood and transferred into another (second) variable domain, thereby substituting the CDRs of the second variable domain. For illustration the CDRs of SEQ ID NO:1 and 24 may be used to replaced the CDRs of SEQ ID NO: 2 and 27. Additionally, the framework of a variable domain which is not shown in Fig. 1 may be used. A variety of variable domains or antibody sequences are known in the art and may be used for this purpose. For example, variable domains, into which CDRs of interest are inserted, may be obtained from any germ-line or rearranged human variable domain. Variable domains may also be synthetically produced. The CDR regions can be introduced into the respective variable domains using recombinant DNA technology. One means by which this can be achieved is described in Marks et al., 1992, Bio/Technology 10:779-783. A variable heavy domain may be paired with a variable light domain to provide an antigen binding site. In addition, independent regions (e.g., a variable heavy domain alone) may be used to bind antigen.

Finally, in another embodiment, the CDRs may be transferred to a non variable domain neighborhood as long as the neighborhood arranges the CDRs to allow for specific binding to RAGE.

In a preferred embodiment of the polypeptide or polypeptide complex of the present invention, the amino acid sequence of SEQ ID NO:1, SEQ ID NO:2, SEQ ID NO:3, SEQ ID NO:4, SEQ ID NO:5, SEQ ID NO:6, SEQ ID NO:7, SEQ ID NO:8, SEQ ID NO.9, SEQ ID NO:10, SEQ ID NO: 11, SEQ ID NO:12, SEQ ID NO:13, SEQ ID NO:14, SEQ ID NO:15, SEQ ID NO:16, SEQ ID NO:17, SEQ ID NO:18, SEQ ID NO:19, SEQ ID NO:20, SEQ ID NO:21, SEQ ID NO:22 and/or SEQ ID NO:23 or a functionally active variant thereof is a variable domain of a light chain (VL).

Alternatively or additionally, the amino acid sequence of SEQ ID NO:24, SEQ ID NO:25, SEQ ID NO:26, SEQ ID NO:27, SEQ ID NO:28, SEQ ID NO:29, SEQ ID NO:30, SEQ ID NO:31, SEQ ID NO:32, SEQ ID NO:33, SEQ ID NO:34, SEQ ID NO:35, SEQ ID NO:36, SEQ ID NO:37, SEQ ID NO:38, SEQ ID NO:39, SEQ ID NO:40, SEQ ID NO:41, SEQ ID NO:42, SEQ ID NO:43, SEQ ID NO:44, SEQ ID NO:45 and/or SEQ ID NO:46 or a functionally active variant thereof is a variable domain of a heavy chain (VH).

In a preferred embodiment of the present invention, the polypeptide or polypeptide complex is an antibody.

Naturally occurring antibodies are globular plasma proteins (~150 kDa) that are also known as immunoglobulins which share a basic structure. As they have sugar chains added to amino acid residues, they are glycoproteins. The basic functional unit of each antibody is an immunoglobulin (Ig) monomer (containing only one Ig unit); secreted antibodies can also be dimeric with two Ig units as with IgA, tetrameric with four Ig units like teleost fish IgM, or pentameric with five Ig units, like mammalian IgM. In the present invention, examples of suitable formats include the format of naturally occurring antibodies including antibody isotypes known as IgA, IgD, IgE, IgG and IgM.

The Ig monomer is a "Y"-shaped molecule that consists of four polypeptide chains; two identical heavy chains and two identical light chains connected by disulfide bonds between cysteine residues. Each heavy chain is about 440 amino acids long; each light chain is about 220 amino acids long. Heavy and light chains each contain intrachain disulfide bonds which stabilize their folding. Each chain is composed of structural domains called Ig domains. These domains contain about 70-110 amino acids and are classified into different categories (for example, variable or V, and constant or C) according to their size and function. They have a characteristic immunoglobulin fold in which two beta sheets create a "sandwich" shape, held together by interactions between conserved cysteines and other charged amino acids.

There are five types of mammalian Ig heavy chain denoted by α, δ, ε, γ, and µ. The type of heavy chain present defines the isotype of antibody; these chains are found in IgA, IgD, IgE, IgG, and IgM antibodies, respectively.

Distinct heavy chains differ in size and composition; α and γ contain approximately 450 amino acids and δ approximately 500 amino acids, while µ and ε have approximately 550 amino acids. Each heavy chain has two regions, the constant region (CH) and the variable region (VH). In one species, the constant region is identical in all antibodies of the same isotype, but differs in antibodies of different isotypes. Heavy chains γ, α and δ have a constant region composed of three tandem Ig domains, and a hinge region for added flexibility; heavy chains µ and ε have a constant region composed of four immunoglobulin domains. The variable region of the heavy chain differs in antibodies produced by different B cells, but is the same for all antibodies produced by a single B cell or B cell clone. The variable region of each heavy chain is approximately 110 amino acids long and is composed of a single Ig domain.

In mammals there are two types of immunoglobulin light chain denoted by λ and κ. A light chain has two successive domains: one constant domain (CL) and one variable domain (VL). The approximate length of a light chain is 211 to 217 amino acids. Each antibody contains two light chains that are always identical; only one type of light chain, κ or λ, is present per antibody in mammals. Other types of light chains, such as the τ chain, are found in lower vertebrates like Chondrichthyes and Teleostei.

In addition to naturally occurring antibodies, artificial antibody formats including antibody fragments have been developed. Some of them are described in the following. However, any other antibody format comprising or consisting of the above polypeptide(s) and allowing for specific binding to RAGE are encompassed by the present invention as well.

Although the general structure of all antibodies is very similar, the unique property of a given antibody is determined by the variable (V) regions, as detailed above. More specifically, variable loops, three each the light (VL) and three on the heavy (VH) chain, are responsible for binding to the antigen, i.e. for its antigen specificity. These loops are referred to as the Complementarity Determining Regions (CDRs). Because CDRs from both VH and VL domains contribute to the antigen-binding site, it is the combination of the heavy and the light chains, and not either alone, that determines the final antigen specificity.

Accordingly, the term "antibody", as used herein, means any polypeptide which has structural similarity to a naturally occurring antibody and is capable of specifically binding to RAGE, wherein the binding specificity is determined by the CDRs of in SEQ ID NOs: 1 to 46, e.g. as shown in Fig. 1. Hence, "antibody" is intended to relate to an immunoglobulin-derived structure with specific binding to RAGE including, but not limited to, a full length or whole antibody, an antigen binding fragment (a fragment derived, physically or conceptually, from an antibody structure), a derivative of any of the foregoing, a chimeric molecule, a fusion of any of the foregoing with another polypeptide, or any alternative structure/composition which selectively binds to RAGE and optionally inhibits the function of RAGE. The antibody may be any polypeptide which comprises at least one antigen binding fragment. Antigen binding fragments consist of at least the variable domain of the heavy chain and the variable domain of the light chain, arranged in a manner that both domains together are able to bind to the specific antigen.

"Full length" or "complete" antibodies refer to proteins that comprise two heavy (H) and two light (L) chains inter-connected by disulfide bonds which comprise: (1) in terms of the heavy chains, a variable region and a heavy chain constant region which comprises three domains, CH1, CH2 and CH3; and (2) in terms of the light chains, a light chain variable region and a light chain constant region which comprises one domain, CL. With regard to the term "complete antibody", any antibody is meant that has a typical overall domain structure of a naturally occurring antibody (i.e. comprising a heavy chain of three or four constant domains and a light chain of one constant domain as well as the respective variable domains), even though each domain may comprise further modifications, such as mutations, deletions, or insertions, which do not change the overall domain structure.

An "antibody fragment" also contains at least one antigen binding fragment as defined above, and exhibits essentially the same function and specificity as the complete antibody of which the fragment is derived from. Limited proteolytic digestion with papain cleaves the Ig prototype into three fragments. Two identical amino terminal fragments, each containing one entire L chain and about half an H chain, are the antigen binding fragments (Fab). The third fragment, similar in size but containing the carboxyl terminal half of both heavy chains with their interchain disulfide bond, is the crystalizable fragment (Fc). The Fc contains carbohydrates, complement-binding, and FcR-binding sites. Limited pepsin digestion yields a single F(ab')2 fragment containing both Fab pieces and the hinge region, including the H-H interchain disulfide bond. F(ab')2 is divalent for antigen binding. The disulfide bond of F(ab')2 may be cleaved in order to obtain Fab'. Moreover, the variable regions of the heavy and light chains can be fused together to form a single chain variable fragment (scFv).

As the first generation of full sized antibodies presented some problems, many of the second generation antibodies have comprised only fragments of the antibody. Variable domains (Fvs) are the smallest fragments with an intact antigen-binding domain consisting of one VL and one VH. Such fragments, with only the binding domains, can be generated by enzymatic approaches or expression of the relevant gene fragments, e.g. in bacterial and eukaryotic cells. Different approaches can be used, e.g. either the Fv fragment alone or 'Fab'-fragments comprising one of the upper arms of the "Y" that includes the Fv plus the first constant domains. These fragments are usually stabilized by introducing a polypeptide link between the two chains which results in the production of a single chain Fv (scFv). Alternatively, disulfide-linked Fv (dsFv) fragments may be used. The binding domains of fragments can be combined with any constant domain in order to produce full length antibodies or can be fused with other proteins and polypeptides.

A recombinant antibody fragment is the single-chain Fv (scFv) fragment. In general, it has a high affinity for its antigen and can be expressed in a variety of hosts. These and other properties make scFv fragments not only applicable in medicine, but also of potential for biotechnological applications. As detailed above, in the scFv fragment the VH and VL domains are joined with a hydrophilic and flexible peptide linker, which improves expression and folding efficiency. Usually linkers of about 15 amino acids are used, of which the (Gly4Ser)3 linker has been used most frequently. scFv molecules might be easily proteolytically degraded, depending on the linker used. With the development of genetic engineering techniques these limitations could be practically overcome by research focussed on improvement of function and stability. An example is the generation of disulfide-stabilized (or disulfide-linked) Fv fragments where the VH-VL dimer is stabilised by an interchain disulfide bond. Cysteines are introduced at the interface between the VL and VH domains, forming a disulfide bridge, which holds the two domains together.

Dissociation of scFvs results in monomeric scFvs, which can be complexed into dimers (diabodies), trimers (triabodies) or larger aggregates such as TandAbs and Flexibodies.

Antibodies with two binding domains can be created either through the binding of two scFv with a simple polypeptide link (scFv)2 or through the dimerisation of two monomers (diabodies). The simplest designs are diabodies that have two functional antigen-binding domains that can be either the same, similar (bivalent diabodies) or have specificity for distinct antigens (bispecific diabodies). These bispecific antibodies allow for example the recruitment of novel effector functions (such as cytotoxic T cells) to the target cells, which make them very useful for applications in medicine.

Recently, antibody formats comprising four variable domains of heavy chains and four variable domains of light chains have been developed. Examples of these include tetravalent bispecific antibodies (TandAbs and Flexibodies, Affimed Therapeutics AG, Heidelberg. Germany). In contrast to a bispecific diabody, a bispecific TandAb is a homodimer consisting of only one polypeptide. Because the two different chains, a diabody can build three different dimers only one of which is functional. Therefore, it is simpler and cheaper to produce and purify this homogeneous product. Moreover, the TandAb usually shows better binding properties (possessing twice the number of binding sites) and increased stability in vivo. Flexibodies are a combination of scFv with a diabody multimer motif resulting in a multivalent molecule with a high degree of flexibility for joining two molecules which are quite distant from each other on the cell surface. If more than two functional antigen-binding domains are present and if they have specificity for distinct antigens, the antibody is multispecific.

In summary, specific immunoglobulins, into which particular disclosed sequences may be inserted or, in the alternative, form the essential part of, include but are not limited to the following antibody molecules which form particular embodiments of the present invention: a Fab (monovalent fragment with variable light (VL), variable heavy (VH), constant light (CL) and constant heavy 1 (CH1) domains), a F(ab')2 (bivalent fragment comprising two Fab fragments linked by a disulfide bridge or alternative at the hinge region), a Fv (VL and VH domains), a scFv (a single chain Fv where VL and VH are joined by a linker, e.g., a peptide linker), a bispecific antibody molecule (an antibody molecule comprising a polypeptide as disclosed herein linked to a second functional moiety having a different binding specificity than the antibody, including, without limitation, another peptide or protein such as an antibody, or receptor ligand), a bispecific single chain Fv dimer, a diabody, a triabody, a tetrabody, a minibody (a scFv joined to a CH3).

Certain antibody molecules including, but not limited to, Fv, scFv, diabody molecules or domain antibodies (Domantis) may be stabilized by incorporating disulfide bridges to line the VH and VL domains. Bispecific antibodies may be produced using conventional technologies, specific methods of which include production chemically, or from hybrid hybridomas) and other technologies including, but not limited to, the BiTETM technology (molecules possessing antigen binding regions of different specificity with a peptide linker) and knobs-into-holes engineering.

Accordingly, the antibody may be a Fab, a Fab', a F(ab')2, a Fv, a disulfide-linked Fv, a scFv, a (scFv)2, a bivalent antibody, a bispecific antibody, a multispecific antibody, a diabody, a triabody, a tetrabody or a minibody.

In another preferred embodiment, the antibody is a monoclonal antibody, a chimeric antibody or a humanised antibody. Monoclonal antibodies are monospecific antibodies that are identical because they are produced by one type of immune cell that are all clones of a single parent cell. A chimeric antibody is an antibody in which at least one region of an immunoglobulin of one species is fused to another region of an immunoglobulin of another species by genetic engineering in order to reduce its immunogenecity. For example murine VL and VH regions may be fused to the remaining part of a human immunoglobulin. A particular type of chimeric antibodies are humanised antibodies. Humanised antibodies are produced by merging the DNA that encodes the CDRs of a non-human antibody with human antibody-producing DNA. The resulting DNA construct can then be used to express and produce antibodies that are usually not as immunogenic as the non-human parenteral antibody or as a chimeric antibody, since merely the CDRs are non-human.

In a preferred embodiment of the present invention, the polypeptide or polypeptide complex comprises a heavy chain immunoglobulin constant domain selected from the group consisting of: a human IgM constant domain, a human IgG1 constant domain, a human IgG2 constant domain, a human IgG3 constant domain, domain, a human IgG4 constant domain, a human IgE constant domain, and a human IgA constant domain.

As detailed above in the context with the antibody of the present invention, each heavy chain of a naturally occurring antibody has two regions, the constant region and the variable region. There are five types of mammalian immunoglobulin heavy chain: γ, δ, α, µ and ε, which define classes of immunoglobulins IgM, IgD, IgG, IgA and IgE, repectively.

There are here are four IgG subclasses (IgGl, 2, 3 and 4) in humans, named in order of their abundance in serum (IgGl being the most abundant). Even though there is about 95 % similarity between their Fc regions of the IgG subclasses, the structure of the hinge regions are relatively different. This region, between the Fab arms (Fragment antigen binding) and the two carboxy-terminal domains CH2 and CH3 of both heavy chains, determines the flexibility of the molecule. The upper hinge (towards the amino-terminal) segment allows variability of the angle between the Fab arms (Fab-Fab flexibility) as well as rotational flexibility of each individual Fab. The flexibility of the lower hinge region (towards the carboxy-terminal) directly determines the position of the Fab-arms relative to the Fc region (Fab-Fc flexibility). Hinge-dependent Fab-Fab and Fab-Fc flexibility may be important in triggering further effector functions such as complement activation and Fc receptor binding. Accordingly, the structure of the hinge regions gives each of the four IgG classes their unique biological profile.

The length and flexibility of the hinge region varies among the IgG subclasses. The hinge region of IgG1 encompasses amino acids 216-231 and since it is freely flexible, the Fab fragments can rotate about their axes of symmetry and move within a sphere centered at the first of two inter-heavy chain disulfide bridges. IgG2 has a shorter hinge than IgG1, with 12 amino acid residues and four disulfide bridges. The hinge region of IgG2 lacks a glycine residue, it is relatively short and contains a rigid poly-proline double helix, stabilised by extra inter-heavy chain disulfide bridges. These properties restrict the flexibility of the IgG2 molecule. IgG3 differs from the other subclasses by its unique extended hinge region (about four times as long as the IgG1 hinge), containing 62 amino acids (including 21 prolines and 11 cysteines), forming an inflexible poly-proline double helix. In IgG3 the Fab fragments are relatively far away from the Fc fragment, giving the molecule a greater flexibility. The elongated hinge in IgG3 is also responsible for its higher molecular weight compared to the other subclasses. The hinge region of IgG4 is shorter than that of IgG1 and its flexibility is intermediate between that of IgG1 and IgG2.

In a preferred embodiment of the present invention, the functionally active variant of any of the above sequences of SEQ OID NO: I to 46 may be used instead of the sequence indicated. For example, the variant may be defined in that the variant
a) is a functionally active fragment consisting of at least 60 %, preferably at least 70 %, more preferably at least 80 %, still more preferably at least 90 %, even more preferably at least 95 %, most preferably 99 % of an amino acid sequence of any of the SEQ ID NOS: I to 46;
b) is a functionally active variant having at least 60 %, preferably at least 70 %, more preferably at least 80 %, still more preferably at least 90 %, even more preferably at least 95 %, most preferably 99 % sequence identity to an amino acid sequence of any of the SEQ ID NOS: 1 to 46; or
c) consists of an amino acid sequence of any of the SEQ ID NOS: 1 to 46 and 1 to 50 additional amino acid residue(s), preferably 1 to 40, more preferably 1 to 30, even more preferably at most 1 to 25, still more preferably at most 1 to 10, most preferably 1, 2, 3, 4 or 5 additional amino acids residue(s).

The fragment as defined in a) is characterized by being derived from any of the sequences of SEQ ID NO: 1 to 46 by one or more deletions. The deletion(s) may be C-terminally, N-terminally and/or internally- Preferably the fragment is obtained by 1, 2, 3, 4, 5, 6, 7, 8, 9 or 10, more preferably 1, 2, 3, 4 or 5, even more preferably 1, 2 or 3, still more preferably 1 or 2, most preferably 1 deletion(s). The functionally active fragment of the invention is characterized by having a biological activity similar to that displayed by the complete protein, including the ability to bind to RAGE, and optionally to inhibit RAGE. The fragment of an antigen is functionally active in the context of the present invention, if the activity of the fragment amounts to at least 10 %, preferably at least 25 %, more preferably at least 50 %, even more preferably at least 70 %, still more preferably at least 80 %, especially at least 90 %, particularly at least 95 %, most preferably at least 99 % of the activity of the antigen without sequence alteration. Suitable methods for determining binding activity to RAGE are given in the Examples.

The variant as defined in b) is characterized by being derived from any of the sequences of SEQ ID NO: 1 to 46 by one or more amino acid modifications including deletions, additions and/or substitutions. The modification(s) may be C-terminally, N-terminally and/or internally. Preferably the fragment is obtained by 1, 2, 3, 4, 5, 6, 7, 8, 9 or 10, more preferably 1, 2, 3, 4 or 5, even more preferably 1, 2 or 3, still more preferably 1 or 2, most preferably 1 modification(s). The functionally active variant of the invention is characterized by having a biological activity similar to that displayed by the complete protein, including the ability to bind to RAGE, and optionally to inhibit RAGE. The fragment of an antigen is functionally active in the context of the present invention, if the activity of the fragment amounts to at least 10 %, preferably at least 25 %, more preferably at least 50 %, even more preferably at least 70 %, still more preferably at least 80 %, especially at least 90 %, particularly at least 95 %, most preferably at least 99 % of the activity of the antigen without sequence alteration.

The variant as defined in c) is characterized in that it consists of an amino acid sequence of any of the SEQ ID NOS: 1 to 46 and 1 to 50 additional amino acid residue(s). The addition(s) may be C-terminally, N-terminally and/or internally. Preferably the variant is obtained by 1, 2, 3, 4, 5, 6, 7, 8, 9 or 10, more preferably 1, 2, 3, 4 or 5, even more preferably 1, 2 or 3, still more preferably 1 or 2, most preferably 1 addition(s). The functionally active variant is further defined as above (see variant of b)).

The additional amino acid residue(s) of (b) and/or (c), may be any amino acid, which may be either an L-and/or a D-amino acid, naturally occurring and otherwise. Preferably, the amino acid is any naturally occurring amino acid such as alanine, cysteine, aspartic acid, glutamic acid, phenylalanine, glycine, histidine, isoleucine, lysine, leucine, methionine, asparagine, proline, glutamine, arginine, serine, threonine, valine, tryptophan or tyrosine.

However, the amino acid may also be a modified or unusual amino acid. Examples of those are 2-aminoadipic acid, 3-aminoadipic acid, beta-alanine, 2-aminobutyric acid, 4-aminobutyric acid, 6-aminocaproic acid, 2-aminoheptanoic acid, 2-aminoisobutyric acid, 3-aminoisobutyric acid, 2-aminopimelic acid, 2,4-diaminobutyric acid, desmosine, 2,2'-diaminopimelic acid, 2,3-diaminopropionic acid, N-ethylglycinem N-ethylasparagine, hydroxylysine, allo-hydroxylysine, 3-hydroxyproloine, 4-hydroxyproloine, isodesmosine, allo-isoleucina, N-methylglycine, N-methylisoleucine, 6-N-Methyllysine, N-methylvaline, norvaline, norleucine or ornithine. Additionally, the amino acid may be subject to modifications such as posttranslational modifications. Examples of modifications include acetylation, amidation, blocking, formylation, □-carboxyglutamic acid hydroxylation, glycosilation, methylation, phosphorylation and sulfatation. If more than one additional or heterologous amino acid residue is present in the peptide, the amino acid residues may be the same or different from one another.

The percentage of sequence identity can be determined e.g. by sequence alignment. Methods of alignment of sequences for comparison are well known in the art. Various programs and alignment algorithms have been described e.g. in Smith and Waterman, Adv. Appl. Math. 2: 482, 1981 or Pearson and Lipman, Proc. Natl. Acad. Sci.US. A. 85: 2444, 1988.

The NCBI Basic Local Alignment Search Tool (BLAST) (Altschul et al., J. Mol. Biol. 215: 403-410, 1990) is available from several sources, including the National Center for Biotechnology Information (NCBI, Bethesda, MD) and on the Internet, for use in connection with the sequence analysis programs blastp, blastn, blastx, tblastn and tblastx. Variants of any of the sequences of SEQ ID NOS: 1 to 46 are typically characterized using the NCBI Blast 2.0, gapped blastp set to default parameters. For comparisons of amino acid sequences of at least 30 amino acids, the Blast 2 sequences function is employed using the default BLOSUM62 matrix set to default parameters, (gap existence cost of 11, and a per residue gap cost of 1). When aligning short peptides (fewer than around 30 amino acids), the alignment is performed using the Blast 2 sequences function, employing the PAM30 matrix set t default parameters (open gap 9, extension gap 1 penalties). Methods for determining sequence identity over such short windows such as 15 amino acids or less are described at the website that is maintained by the National Center for Biotechnology Information in Bethesda, Maryland.

In a more preferred embodiment the functionally active variant, as defined above, is derived from the amino acid sequence of any of the SEQ ID NOS: 1 to 46 of any of the SEQ ID NOS: 1 to 46 by one or more conservative amino acid substitution.

Conservative amino acid substitutions, as one of ordinary skill in the art will appreciate, are substitutions that replace an amino acid residue with one imparting similar or better (for the intended purpose) functional and/or chemical characteristics. For example, conservative amino acid substitutions are often ones in which the amino acid residue is replaced with an amino acid residue having a similar side chain. Families of amino acid residues having similar side chains have been defined in the art. These families include amino acids with basic side chains (e.g., lysine, arginine, histidine), acidic side chains (e.g., aspartic acid, glutamic acid), uncharged polar side chains (e.g., glycine, asparagine, glutamine, serine, threonine, tyrosine, cysteine, tryptophan), nonpolar side chains (e.g., alanine, valine, leucine, isoleucine, proline, phenylalanine, methionine), beta-branched side chains (e.g., threonine, valine, isoleucine) and aromatic side chains (e.g., tyrosine, phenylalanine, tryptophan, histidine). Such modifications are not designed to significantly reduce or alter the binding or functional inhibition characteristics of the polypeptide (complex), albeit they may improve such properties. The purpose for making a substitution is not significant and can include, but is by no means limited to, replacing a residue with one better able to maintain or enhance the structure of the molecule, the charge or hydrophobicity of the molecule, or the size of the molecule. For instance, one may desire simply to substitute a less desired residue with one of the same polarity or charge. Such modifications can be introduced by standard techniques known in the art, such as site-directed mutagenesis and PCR-mediated mutagenesis. One specific means by which those of skill in the art accomplish conservative amino acid substitutions is alanine scanning mutagenesis. The altered polypeptides are then tested for retained or better function using functional assays available in the art or described in the Examples. In a more preferred embodiment of the present invention the number of conservative substitutions in any of the sequences of SEQ ID NO: 1 to 46 is at most 20, 19, 18, 27, 26, 15, 14, 13, 12 or 11, preferably at most 10, 9, 8, 7 or 6, especially at most 5, 4, 3 particularly 2 or 1.

Another aspect of the present invention relates to one or more nucleic acid(s) coding for the polypeptide or polypeptide complex according to the present invention. Nucleic acid molecules of the present invention may be in the form of RNA, such as mRNA or RNA, or in the form of DNA, including, for instance, cDNA and genomic DNA e.g. obtained by cloning or produced by chemical synthetic techniques or by a combination thereof. The DNA may be triple-stranded, double- stranded or single-stranded. Single-stranded DNA may be the coding strand, also known as the sense strand, or it may be the non-coding strand, also referred to as the anti-sense strand. Nucleic acid molecule as used herein also refers to, among other, single- and double- stranded DNA, DNA that is a mixture of single- and double-stranded RNA, and RNA that is a mixture of single- and double-stranded regions, hybrid molecules comprising DNA and RNA that may be single-stranded or, more typically, double-stranded, or triple-stranded, or a mixture of single-and double-stranded regions. In addition, nucleic acid molecule as used herein refers to triple-stranded regions comprising RNA or DNA or both RNA and DNA.

The nucleic acid also includes sequences that are a result of the degeneration of the genetic code. There are 20 natural amino acids, most of which are specified by more than one codon. Therefore, all nucleotide sequences are included in the invention which result in the peptide(s) as defined above.

Additionally, the nucleic acid may contain one or more modified bases. Such nucleic acids may also contain modifications e.g. in the ribose-phosphate backbone to increase stability and half life of such molecules in physiological environments. Thus, DNAs or RNAs with backbones modified for stability or for other reasons are "nucleic acid molecule" as that feature is intended herein. Moreover, DNAs or RNAs comprising unusual bases, such as inosine, or modified bases, such as tritylated bases, to name just two examples, are nucleic acid molecule within the context of the present invention. It will be appreciated that a great variety of modifications have been made to DNA and RNA that serve many useful purposes known to those of skill in the art. The term nucleic acid molecule as it is employed herein embraces such chemically, enzymatically or metabolically modified forms of nucleic acid molecule, as well as the chemical forms of DNA and RNA characteristic of viruses and cells, including simple and complex cells, inter alia. For example, nucleotide substitutions can be made which do not affect the polypeptide encoded by the nucleic acid, and thus any nucleic acid molecule which encodes an antigen or fragment or functional active variant thereof as defined above is encompassed by the present invention.

Furthermore, any of the nucleic acid molecules encoding one or more polypeptides of the invention including fragments or functionally active variants thereof can be functionally linked, using standard techniques such as standard cloning techniques, to any desired regulatory sequence, leader sequence, heterologous marker sequence or a heterologous coding sequence to create a fusion protein.

The nucleic acid of the invention may be originally formed in vitro or in a cell in culture, in general, by the manipulation of nucleic acids by endonucleases and/or exonucleases and/or polymerases and/or ligases and/or recombinases or other methods known to the skilled practitioner to produce the nucleic acids.

In a preferred embodiment, the nucleic acid(s) is/are located in a vector. A vector may additionally include nucleic acid sequences that permit it to replicate in the host cell, such as an origin of replication, one or more therapeutic genes and/or selectable marker genes and other genetic elements known in the art such as regulatory elements directing transcription, translation and/or secretion of the encoded protein. The vector may be used to transduce, transform or infect a cell, thereby causing the cell to express nucleic acids and/or proteins other than those native to the cell. The vector optionally includes materials to aid in achieving entry of the nucleic acid into the cell, such as a viral particle, liposome, protein coating or the like. Numerous types of appropriate expression vectors are known in the art for protein expression, by standard molecular biology techniques. Such vectors are selected from among conventional vector types including insects, e.g., baculovirus expression, or yeast, fungal, bacterial or viral expression systems. Other appropriate expression vectors, of which numerous types are known in the art, can also be used for this purpose. Methods for obtaining such expression vectors are well-known (see, e.g. Sambrook et al, Molecular Cloning. A Laboratory Manual, 2d edition, Cold Spring Harbor Laboratory, New York (1989)). In one embodiment, the vector is a viral vector. Viral vectors include, but are not limited to, retroviral and adenoviral vectors.

Suitable host cells or cell lines for transfection by this method include bacterial cells. For example, the various strains of E. coli are well-known as host cells in the field of biotechnology. Various strains of B. subtilis, Pseudomonas, Streptomyces, and other bacilli and the like may also be employed in this method. Many strains of yeast cells known to those skilled in the art are also available as host cells for expression of the peptides of the present invention. Other fungal cells or insect cells such as Spodoptera frugipedera (Sf9) cells may also be employed as expression systems. Alternatively, mammalian cells, such as human 293 cells, Chinese hamster ovary cells (CHO), the monkey COS-1 cell line or murine 3T3 cells derived from Swiss, BALB/c or NIH mice may be used. Still other suitable host cells, as well as methods for transfection, culture, amplification, screening, production, and purification are known in the art.

A polypeptide(s) or polypeptide complex of the invention may be produced by expressing a nucleic acid of the invention in a suitable host cell. The host cells can be transfected, e.g. by conventional means such as electroporation with at least one expression vector containing a nucleic acid of the invention under the control of a transcriptional regulatory sequence. The transfected or transformed host cell is then cultured under conditions that allow expression of the protein. The expressed protein is recovered, isolated, and optionally purified from the cell (or from the culture medium, if expressed extracellularly) by appropriate means known to one of skill in the art. For example, the proteins are isolated in soluble form following cell lysis, or extracted using known techniques, e.g. in guanidine chloride. If desired, the polypeptide(s) of the invention are produced as a fusion protein. Such fusion proteins are those described above. Alternatively, for example, it may be desirable to produce fusion proteins to enhance expression of the protein in a selected host cell or to improve purification. The molecules comprising the polypeptides of this invention may be further purified using any of a variety of conventional methods including, but not limited to: liquid chromatography such as normal or reversed phase, using HPLC, FPLC and the like; affinity chromatography (such as with inorganic ligands or monoclonal antibodies); size exclusion chromatography; immobilized metal chelate chromatography; gel electrophoresis; and the like. One of skill in the art may select the most appropriate isolation and purification techniques without departing from the scope of this invention. Such purification provides the antigen in a form substantially free from other proteinaceous and non-proteinaceous materials of the microorganism.

Another aspect of the present invention relates to a cell producing an antibody according to the present invention.

As other proteins the polypeptides of the present invention may be produced in vitro in a series of cellular expression systems. These may include CHO cells (derived from Chinese Hamster Ovaries), yeasts (Saccharomyces or Pichia), filamentous fungi, transgenic plants, and E. coli.

In the beginning, the use of E. coli expression systems has been limited mainly to the production of antibody fragments These fragments have been successfully expressed and secreted in E. coli. Fabs are frequently used in diagnostic applications, therapeutics, and in testing variable regions slated for reincorporation into full-length monoclonal antibodies. Another successful application in E. coli production is the fusion of a functional protein with a Fab. The antigen targeting-specific Fab region is fused to a functional protein sequence. Creating targeted therapeutics with enhanced cell killing is one application of this approach. Other strategies involving antibody fragments include, fusing target specific protein domains such as receptor fragments to Fc (receptor-binding fragment) regions, The Fc fragment of the antibody is responsible for the long serum half-life along with activation of the immune system. Applications of Fc fusions depend on combining the binding activity of the fusion partner with the activation of the Fc region. However, production of the Fc region in E. coli can be problematic due to the difficulty of effectively expressing the Fc fragment in bacteria. This may explain why production of the full monoclonal antibody in E. coli has also remained an elusive goal. As described below, improved methods for expression of both fragments and full monoclonal antibodies in E. coli have been developed. While the mammalian cell machinery is crucial for antibody production attributes such as glycosylation, many opportunities exist for an effective E. coli antibody production system. Translation Engineering has been used to optimize genes for expression of antibodies and antibody fragments effectively in E. coli. Translation Engineering includes industry standard techniques such as removing rare codons, smoothing out RNA secondary structure, identification and manipulation of translation pause signals that effect the step-wise kinetics of the ribosome while it is translating the antibody mRNA. After manipulation of the gene coding the antibody of interest, the redesigned gene construct is put into an appropriate vector which may include both heavy and light chain components.

In another embodiment the cell is a hybridoma cell lines expressing desirable monoclonal antibodies are generated by well-known conventional techniques. In the context of the present invention the hybridoma cell is able to produce an antibody specifically binding to RAGE. The hybridoma cell can be generated by fusing a normal-activated, antibody-producing B cell with a myeloma cell. In particular, the hybrodoma cell may be produced as follows: B-cells are removed from the spleen of an animal that has been challenged with the relevant antigen. These B-cells are then fused with myeloma tumor cells that can grow indefinitely in culture. This fusion is performed by making the cell membranes more permeable. The fused hybrid cells (called hybridomas), being cancer cells, will multiply rapidly and indefinitely and will produce large amounts of the desired antibodies. They have to be selected and subsequently cloned by limiting dilution. Supplemental media containing Interleukin-6 (such as briclone) are usually essential for this step. Selection occurs via culturing the newly fused primary hybridoma cells in selective-media, specifically media containing 1 x concentration HAT for roughly 10-14 days. After using HAT it is often desirable to use HT containing media. Cloning occurs after identification of positive primary hybridoma cells.

Another aspect of the present invention relates to a binding molecule capable of binding to RAGE and comprising the polypeptide or polypeptide complex according to the invention. The polypeptides (or complexes thereof) and antibodies of the present invention may be used in a variety of applications including medicine, therapy, diagnosis, but also science and research, e.g. for detection, purification, labeling etc.

Accordingly, it may be necessary to add a further component to the polypeptide (complex) of the present invention. Particularly, it may be desirable, to add a marker for detection of the molecule to the same. Suitable markers include without limitation a tag (e.g. 6 His (or HexaHis) tag, Strep tag, HA tag, c-myc tag or glutathione S-transferase (GST) tag), fluorescence marker (e.g. FITC, fluorescein, rhodamine, Cy dyes or Alexa), enzyme label (e.g. penicillinase, horseradish peroxidase and alkaline phosphatase), a radiolabel (e.g. 3H, 32P, 35S, 1251 or 14C). Additionally, the polypeptide (complex) may be add to a support, particularly a solid support such as an array, bead (e.g. glass or magnetic), a fiber, a film etc. The skilled person will be able to adapt the binding molecule comprising the polypeptide or polypeptide complex of the present invention and a further component to the intended use by choosing a suitable further component.

Another aspect of the present invention relates to a composition for use as a medicament, the composition comprising at least one polypeptide of the invention and/or at least one nucleic acid of the invention.

The pharmaceutical composition of the present invention may further encompass pharmaceutically acceptable carriers and/or excipients. The pharmaceutically acceptable carriers and/or excipients useful in this invention are conventional and may include buffers, stabilizers, diluents, preservatives, and solubilizers. Remington's Pharmaceutical Sciences, by E. W. Martin, Mack Publishing Co., Easton, PA, 15th Edition (1975), describes compositions and formulations suitable for pharmaceutical delivery of the polypeptides/nucleic acids disclosed herein. The content of the active ingredient (polypeptide or nucleic acid) in the pharmaceutical composition is not limited as far as it is useful for treating or preventing, but preferably contains 0.0000001-10% by weight per total composition.

In general, the nature of the carrier or excipients will depend on the particular mode of administration being employed. For instance, parenteral formulations usually comprise injectable fluids that include pharmaceutically and physiologieally acceptable fluids such as water, physiological saline, balanced salt solutions, aqueous dextrose, glycerol or the like as a vehicle. For solid compositions (e. g. powder, pill, tablet, or capsule forms), conventional non-toxic solid carriers can include, for example, pharmaceutical grades of mannitol, lactose, starch, or magnesium stearate. In addition to biologically neutral carriers, pharmaceutical compositions to be administered can contain minor amounts of non-toxic auxiliary substances, such as wetting or emulsifying agents, preservatives, and pH buffering agents and the like, for example sodium acetate or sorbitan monolaurate.

Generally, an appropriate amount of a pharmaceutically acceptable salt is used in the carrier to render the formulation isotonic. Examples of the carrier include but are not limited to saline, Ringer's solution and dextrose solution. Preferably, acceptable excipients, carriers, or stabilisers are preferably non-toxic at the dosages and concentrations employed, including buffers such as citrate, phosphate, and other organic acids; salt-forming counter-ions, e.g. sodium and potassium; low molecular weight (> 10 amino acid residues) polypeptides; proteins, e.g. serum albumin, or gelatine; hydrophilic polymers, e.g. polyvinylpyrrolidone; amino acids such as histidine, glutamine, lysine, asparagine, arginine, or glycine; carbohydrates including glucose, mannose, or dextrins; monosaccharides; disaccharides; other sugars, e.g. sucrose, mannitol, trehalose or sorbitol; chelating agents, e.g. EDTA; non-ionic surfactants, e.g. Tween, Pluronics or polyethylene glycol; antioxidants including methionine, ascorbic acid and tocopherol; and/or preservatives, e.g. octadecyldimethylbenzyl ammonium chloride; hexamethonium chloride; benzalkonium chloride, benzethonium chloride; phenol, butyl or benzyl alcohol; alkyl parabens, e.g. methyl or propyl paraben; catechol; resorcinol; cyclohexanol; 3-pentanol; and m-cresol).

In a preferred embodiment the pharmaceutical composition further comprises an immunostimulatory substance such as an adjuvant. The adjuvant can be selected based on the method of administration and may include mineral oil-based adjuvants such as Freund's complete and incomplete adjuvant, Montanide incomplete Seppic adjuvant such as ISA, oil in water emulsion adjuvants such as the Ribi adjuvant system, syntax adjuvant formulation containingmuramyl dipeptide, or aluminum salt adjuvants. Preferably, the adjuvant is a mineral oil-based adjuvant, most preferably ISA206 (SEPPIC, Paris, France). In a more preferred embodiment the immunostimulatory substance is selected from the group comprising polycationic polymers, especially polycationic peptides such as polyarginine, innmunostimulatory deoxynucleotides (ODNs), peptides containing at least two LysLeuLys motifs, especially KLKLLLLLKLK (SEQ ID NO: 51), neuroactive compounds, especially human growth hormone, alumn, adjuvants or combinations thereof. Preferably the combination is either a polycationic polymer and immunostimulatory deoxynucleotides or of a peptide containing at least two LysLeuLys motifs and immunostimulatory deoxynucleotides. In a still more preferred embodiment the polycationic polymer is a polycationic peptide. In an even more preferred embodiment of the invention the immunostimulatory substance is at least one immunostimulatory nucleic acid. Immunostimulatory nucleic acids are e.g. neutral or artificial CpG containing nucleic acids, short stretches of nucleic acids derived from non-vertebrates or in form of short oligonucleotides (ODNs) containing non-methylated cytosine-guanine dinucleotides (CpG) in a defined base context (e.g. as described in WO 96/02555). Alternatively, also nucleic acids based on inosine and cytidine as e.g. described in WO 01/93903, or deoxynucleic acids containing deoxy-inosine and/or deoxyuridine residues (described in WO 01/93905 and WO 02/095027) may preferably be used as immunostimulatory nucleic acids in the present invention. Preferably, mixtures of different immunostimulatory nucleic acids are used in the present invention. Additionally, the aforementioned polycationic compounds may be combined with any of the immunostimulatory nucleic acids as aforementioned. Preferably, such combinations are according to the ones described in WO 01/93905, WO 02/32451, WO 01/54720, WO 01/93903, WO 02/13857 and WO 02/095027 and the Australian patent application A 1924/2001.

The pharmaceutical composition encompasses at least one polypeptide or nucleic acid of the invention; however, it may also contain a cocktail (i.e., a simple mixture) containing different polypeptides and/or nucleic acids of the invention. The polypeptide(s) of the present invention may also be used in the form of a pharmaceutically acceptable salt. Suitable acids and bases which are capable of forming salts with the peptides of the present invention are well known to those of skill in the art, and include inorganic and organic acids and bases.

In a preferred embodiment of the present invention, the composition is intended or used for treating a RAGE-related disease or disorder as known to the skilled person or as defined herein, preferably selected from the group consisting of sepsis, septic shock, listeriosis, inflammatory disease, including rheumatoid and psoriatic arthritis and intestinal bowel disease, cancer, arthritis, Crohn's disease, chronic acute inflammatory disease, cardiovascular disease, erectile dysfunction, diabetes, complication of diabetes, vasculitis, nephropathy, retinopathy, neuropathy, amyloidoses, atherosclerosis, peripheral vascular disease, myocardial infarction, congestive heart failure, diabetic retinopathy, diabetic neuropathy, diabetic nephropathy and Alzheimer's disease, especially diabetes and/or an inflammatory disorder.

Another aspect of the present invention relates to a method of diagnosing a RAGE-related disease or disorder as defined above, comprising the steps of:
(a) contacting a sample obtained from a subject with the polypeptide or polypeptide complex or a binding molecule according to the present invention; and
(b) detecting the amount of RAGE,
wherein an altered amount of RAGE receptor relative to a control is indicative of a RAGE-related disease or disorder.

The present invention also relates to diagnostic assays such as quantitative and diagnostic assays for detecting RAGE or RAGE levels with the polypeptides or binding of the present invention in cells and tissues or body fluids, including determination of normal and abnormal levels. Assay techniques that can be used to determine levels of a polypeptide or an antibody, in a sample derived from a host are well known to those of skill in the art. Such assay methods include radioimmunoassays, competitive-binding assays, Western Blot analysis and ELISA assays. Among these, ELISAs frequently are preferred. An ELISA assay initially comprises preparing an antibody specific to the polypeptide, particularly RAGE, preferably a monoclonal antibody. In addition, a reporter antibody generally is prepared which binds to the monoclonal antibody. The reporter antibody is attached to a detectable reagent such as radioactive, fluorescent or enzymatic reagent, such as horseradish peroxidase enzyme.

The invention is not limited to the particular methodology, protocols, and reagents described herein because they may vary. Further, the terminology used herein is for the purpose of describing particular embodiments only and is not intended to limit the scope of the present invention. As used herein and in the appended claims, the singular forms "a", "an", and "the" include plural reference unless the context clearly dictates otherwise. Similarly, the words "comprise", "contain" and "encompass" are to be interpreted inclusively rather than exclusively,

Unless defined otherwise, all technical and scientific terms and any acronyms used herein have the same meanings as commonly understood by one of ordinary skill in the art in the field of the invention. Although any methods and materials similar or equivalent to those described herein can be used in the practice of the present invention, the preferred methods, and materials are described herein.

The invention is further illustrated by the following example, although it will be understood that the examples are included merely for purposes of illustration and are not intended to limit the scope of the invention unless otherwise specifically indicated.

### FIGURES:

- Figure 1:: Variable regions of light chain (left) and heavy chain (right) with CDRs.
- Figure 2:: ka/kd of antiRage Hybridomas tested with LP08062 (Rage) at 15 nM.
- Figure 3:: Kd ranges of selected anti-RAGE monoclonal Antibodies
- Figure 4:: Inhibition of RAGE S100A6 interaction.
- Figure 5:: Biacore analysis of RAGE 513_LP08062 (top) and 501-4 RAGE-1 050908 b

### EXAMPLES:

### EXAMPLE 1: Generation and Identification of Antibodies

Generation and Identification of Antibodies has benn achieved according to methods well known to a person skilled in the art. Such methods are disclosed in e.g (i) Handbook of therapeutic antibodiesWiley-VCH, Weinheim; ISBN-10:3-527-31453-9; ISBN-13:978-3-527-31453-9-; and/or in (ii) Therapeutic monoclonal antibodies: from bench to clinic; ISBN: 978-0-470-11791-0; and/or in (iii) Current protocols in Immunology; John Wiley and Sons, Inc.; last updated 1 October 2009.

### EXAMPLE 2: Selection of Advantageous Antibodies

Of the antibodies available "top 23" antibodies have been identified and selected based on
- the binding constants (KD ≤ 1.0 E-9 M and koff ≤ 2.0 E-3 s-1) and
- Cross species reactivity with rat, mouse & Cyno sRAGE

The following amino acid sequences for variable regions of anti-rage monoclonal antibodies have been determined:
Protein 56 RAGE-1
   VL 56: variable region for light chain; entire molecule length: 107 aa; SEQ ID NO: 1
      1 divmtqsqkf mstsvgdxvs vtckasqnvg invawyqqkp gqspkaliys
      51 asyrysgvpd rftgsgsgtd ftliisnvqs edlaeyfcqq ynnyprtfgg
      101 gtkleik
   VH 56: variable region for heavy chain; entire molecule length: 115 aa; SEQ ID NO: 24
      1 qvqlqqsgpe lvkpgasvri sckasgytft syfihwvkqr pgqglewigw
      51 iypgnvntky nekFkdkatl tadkssstay mqlsnltsed savyfcvrgq
      101 lgdywgqgit Itvss
Protein 95 RAGE-1
   VL 95: variable region for light chain; entire molecule length: 109 aa; SEQ ID NO: 2
      1 qavvtqesal ttspgetvtl tcrsstgavt tsnyanwvqe kpdhlftglt
      51 ggtnnrapgv parfsgslig dkaaltitga qtedeaiyfc alwysnhwvf
      101 gggtkltvl
   VH 95: variable region for heavy chain; entire molecule length: 115 aa; SEQ ID NO: 25
      1 qvqlqqpgae lvkpgasvkl sckasgytft sywmhwvkqr pgqglewige
      51 snpsngrtny nekfknkatl tvdkssstay mqlssltsed savyycarap
      101 yygfdywgqg ttltvss
Protein 130 RAGE-1
   VL 130: variable region for light chain; entire molecule length: 106 aa; SEQ ID NO: 3
      1 qivltqspai msaspgekvt mtcsasssvs ymhwyqqksg tspkrwisdt
      51 sklasgvpar fsgsgsgtsy sltissmeae daatyycqqw ssnpptfggg
      101 tkleik
   VH 130: variable region for heavy chain; entire molecule length: 119 aa; SEQ ID NO: 26
      1 evqlvesggg lvkpggslkl scaasgftfs syvmswvrqs pekrlewvae
      51 issggsytyy pdtvtgrfti srdndkntly lemsslrsed tamyycarpp
      101 ygkdamdywg qgtsvtvss
Protein 140 RAGE-1
   VL 140: variable region for light chain; entire molecule length: 108 aa; SEQ ID NO: 4
      1 qivltqspai msaspgekvt iscsasssvs ymywyqqkpg sspkpwiyrt
      51 snlasgvpar fsgsgsgtsy sltissmeae daatyycqqy hsyppmytfg
      101 ggtkleik
   VH 140: variable region for heavy chain; entire molecule length: 121 aa; SEQ ID NO: 27
      1 qvqlqqpgae lvkpgasvrl sckasgytft sywmhwvkqr pgqglewige
      51 inpsngrtny nekfkskatl tvdkssstay mqlssltsed savyycardg
      101 lgyrpiamdy w gqgtsvtvs s
Protein 152 RAGE-1
   VL 152: variable region for light chain; entire molecule length: 110 aa; SEQ ID NO: 5
      1 divltqspas lavslgqrat iscrasksvg tsdssymhwy qqkpgqppkl
      51 hylasnles gvparfsgsg sgtdftlnih pveeedaaty ycqhsrelyt
      101 fgagtkleik
   VH 152: variable region for heavy chain; entire molecule length: 115 aa; SEQ ID NO: 28
      1 dvqlqesgpd lvkpsqslsl tctvtgysit sgyswhwirq fpgnklewmg
      51 yihysgstny npslkstisi trdtsknqff lqlnsvtted tatyycargg
      101 dfaywgqgtl vtvsa
Protein 158 RAGE-1
   VL 158: variable region for light chain; entire molecule length: 113 aa; SEQ ID NO: 6
      1 sdwltqtpl slpvnigdqa sisckstksl Insdgftyld wylqkpgqsp
      51 qlliylvsnr fsgvpdrfsg sgsgtdftlk isrveaedlg vyycfqsnyl
      101 pltfgggtkv eik
   VH 158: variable region for heavy chain; entire molecule length: 119 aa; SEQ ID NO: 29
      1 qiqlvqsgpe lkkpgetvki sckasgytft dysmhwvkqa pgkglkwmgw
      51 intetgepty addfkgrfaf sletsastay llinnlkted tatyfcardy
      101 lyyyamdywg qgtsvtvss
Protein 164 RAGE-1
   VL 164: variable region for light chain; entire molecule length: 107 aa; SEQ ID NO: 7
      1 nivmtqspks msmsvgervt lsckasenvg tyvswyqqkp eqspklliyg
      51 asnrytgvpd rftgsgsatd ftltissvqa edladyhcgq sytypytfgg
      101 gtkleik
   VH 164: variable region for heavy chain; entire molecule length: 116 aa; SEQ ID NO: 30
      1 qvqlqqpgse lvrpgasvkl sckasgytft nywmhwvkqr pgqglewign
      51 iypgsgstny dekfkskatl tvdtssstay mqlssltsed savyyctrlr
      101 rgiaywgqgt lvtvsa
Protein 166 RAGE-1
   VL 166: variable region for light chain; entire molecule length: 112 aa; SEQ ID NO: 8
      1 nimmtqspss lavsagekvt msckssqsvl yssnqknyla wyqqkpgqsp
      51 klliywastr esgvpdrftg sgsgtdftlt issvqaedla vyychqylss
      101 ytfgggtkle ik
   VH 166: variable region for heavy chain; entire molecule length: 119 aa; SEQ ID NO: 31
      1 qvqlqqsgpe lvkpgtsvri sckasgytft syyihwvkqr pgqglewigw
      51 iypgnvitny hekfkgkasl tadkssstay mqlssltsed savyfcared
      101 pfaywgqgtl vtvsa
Protein 173 RAGE-1
   VL 173: variable region for light chain; entire molecule length: 107 aa; SEQ ID NO: 9
      1 divmtqsqkfmstsvgdrvs vtckasqnvg tnvawyqqkp gqspkaliys 51 asyrysgvpd rftgsgsgtd ftltisnvqs edlaeyfcqq ynsypltfga
      101 gtklelk
   VH 173: variable region for heavy chain; entire molecule length: 120 aa; SEQ ID NO: 32
      1 evkleesggg lvqpggsmkl scvasgftfs nywmnwvrqs pekglewvae
      51 irlksnnyat hyaesvkgrf tisrddskss vylqmndlra edpgiyycir
      101 dygnyamdhw gqgtsvtvss
Protein 183 RAGE-1
   VL 183: variable region for light chain; entire molecule length: 107 aa; SEQ ID NO: 10
      1 nivmtqspks msmsvgervt Isckasenvg tyvswyqqkp eqspklliyg
      51 asnrytgvpd rftgsgsatd ftltissvqa edladyhcgq sysypytfgg
      101 gtkleik
   VH 183: variable region for heavy chain; entire molecule length: 116 aa; SEQ ID NO: 33
      1 evqlqqsgtv larpgasvkm sckasgysft sywmhwvkqr pgqglewiga
      51 ifpgnsdtty nqkfkgkakl tavtsastay melssltned savyyctglr
      101 rgfpywgqgt lvtvsv
Protein 184 RAGE-1
   VL 184: variable region for light chain; entire molecule length: 111 aa; SEQ ID NO: 11
      1 divltqspas lavslgqrat iscrasksvs tsgysymhwy qqkpgqppkl
      51 liylashles gvpazfsgsg sgtdfslnih pveeedaaty ycqhsrelpw
      101 tfgggtklei k
   VH 184: variable region for heavy chain; entire molecule length: 120 aa; SEQ ID NO: 34
      1 qvqlqqsgae lvrpgtsvkv sckasgyaft nyliewvkqr pgqglewigm
      51 inpgsggtny nekfkgkatl tadkssstay mqlssltsdd savyfcargr
      101 gghyryfdvw gagttvtvss
Protein 210 RAGE-1
   VL 210: variable region for light chain; entire molecule length: 110 aa; SEQ ID NO: 12
      1 qavvtqesal ttspgetvtl tcrsstgavt tsnyanwvqe kpdhlftgli
      51 ggtnnrapgv parfsgslig dkaaltitga qtedeaiyfc alwysnhfwv
      101 fgggtkltvl
   VH 210: variable region for heavy chain; entire molecule length: 119 aa; SEQ ID NO: 35
      1 hseiqlqqtg pelvkpgasv kisckasgys ftdyimvwvk qshgkslewi
      51 gtinpyygst synlkfkgka tltvdkssst anmqlnslts edsavyycar
      101 lrlyamdywg qgtsvtvss
Protein 240 RAGE-1
   VL 240: variable region for light chain; entire molecule length: 113 aa; SEQ ID NO: 13

      1 sdvvltqtpl slpvsigdqa sisckstksl lnsdgftyld wylqkpgqsp
      51 qlliylvsnr fsgvpdsfsg sgsgtdftlk isrveaedlg vyycfqsnyf
      101 pltfgggttl eik
   VH 240: variable region for heavy chain; entire molecule length: 119 aa; SEQ ID NO: 36
      1 qiqlvqsgpe lkkpgetvki sckasgytft dysmhwvkqa pgkglkwmgw
      51 intetgepty addfkgrfaf sletsastay lqinnlkned tatyfcardy
      101 lyyyamdywg qgtsvtvss
Protein 250 RAGE-1
   VL 250: variable region for light chain; entire molecule length: 107 aa; SEQ ID NO: 14
      1 divmtqsqkf mstsvgdrvs vtckasqnvg tnvawyqqkp gqspkaliys
      51 asyrysgvpd rftgsgsgtd ftltisnvqs edlaeffcqq ynsypltfga
      101 gtklelk
   VH 250: variable region for heavy chain; entire molecule length: 120 aa; SEQ ID NO: 37
      1 evkleesggg lvqpggsmkl scvasgftfs nyvmnwvrqs pekglewvae
      51 irlksnnyat hyaesvkgrf tisrddskss vylqmnnlra edtgiyfcir
      101 dygnyamdyw gqgtsvtvss
Protein 2 53 RAGE-1
   VL 253: variable region for light chain; entire molecule length: 113 aa; SEQ ID NO: 15
      1 divmsqspss lavsvgekvt msckssqtll yssnqknyla wyqqkpgqsl
      51 klliywastr esgvpdrfag sgsgtdftlt issvkaedla vyycqqyfgy
      101 pytfgggikl eik
   VH 253: variable region for heavy chain; entire molecule length: 118 aa; SEQ ID NO: 38
      1 qvqlqqsgpe lvkpgasvri sckasgytft dyyihwvkqr pgqglewigw
      51 iypgnvitky nekfkgkatl tadkssstay mqlssltsed savyfcaryd
      101 ydyamdywgq gtsvtvss
Protein 259 RAGE-1
   VL 259: variable region for light chain; entire molecule length: 109 aa; SEQ ID NO:16
      1 qavvtqesal ttspgetvtl tcrsstgavt tsnyanwvqe kpdhlftgli
      51 ggtrmrapgv parfsgslig dkaaltitga qtedeaiyfc alwysnhwvf
      101 gggtkltvl
   VH 259: variable region for heavy chain; entire molecule length: 117 aa; SEQ ID NO: 39
      1 qvqlqqsgae lvrpgtsvkv sckasgyaft nylidwvnqr pgqglewigv
      51 inpgsggtny nekftgkatl tadkssstay mqlssltsdd savyfcam
   101 vdtmdywgqg tsvtvss
Protein 283 RAGE-1
   VL 283: variable region for light chain; entire molecule length: 109 aa; SEQ ID NO: 17
      1 qavvtqesal ttspgetvt1 tcrsstgavt tsnyanwvqe kpdhlftgli
      51 rgtnnrapgv parfsgslig dkaaltitga qtedeaiyfc alwysnhwvf
      101 gggtkltvl
   VH 283: variable region for heavy chain; entire molecule length: 118 aa; SEQ ID NO: 40
      1 qvqlqqsgae lvrpgtsvkv sckasgyaft nyliewvkqr pgqglewigv
      51 inpgsggtny serfkgkatl tadkssstay mqlssltsdd savyfcasyr

      101 ydggmdywgq gtsvtvss
Protein 316 RAGE-1
   VL 316: variable region for light chain; entire molecule length: 111 aa; SEQ ID NO: 18
      1 divltqspas lavslgqrat iscrasksvs isgysylhwn qqkpgqspkl
      51 liylasnles gvparfsgsg sgtdftlnih pveeedaaty ycqhsrelpy
      101 tfgggtklei k
   VH 316: variable region for heavy chain; entire molecule length: 118 aa; SEQ ID NO: 41
      1 qvqlqqsgpe lvrpgasvkm sckasgytft sywmhwvkqr pgqglewigm
      51 idpsnsetrl nqkfkdkatl nvdkssntay mqlssltsed savyycamf

      101 ygsslrvwga gttvtvss
Protein 326 RAGE-1
   VL 326: variable region for light chain; entire molecule length: 108 aa; SEQ ID NO: 19
      1 divmtqsqkf mstsvgdrvs itckasqnvg tavawyqqkp gqspklliys
      51 asnrytgvpd rftgsgsgtd ftltisnmqs edladyfcqq yssyplltfg
      101 agtklelk
   VH 326: variable region for heavy chain; entire molecule length: 119 aa; SEQ ID NO: 42
      1 evklvesggg lvkpggslkl scaasgfafs sydmswvrqt pekrlewvat
      51 issggsytsy pdsvqgrfti srdnamtly lqmsslrsed talyycassq
      101 lppyamdywg qgtsvtvss
Protein 347 RAGE-1
   VL 347: variable region for light chain; entire molecule length: 107 aa; SEQ ID NO: 20
      1 diqmtqsssy lsvslggrvt itckasdrin ywlawyqqkp gnaprllisg
      51 attletgvps rfsgsgsgkd ytlsitslqt edvatyycqq ywstpytfgg
      101 gtklelk
   VH 347: variable region for heavy chain; entire molecule length: 118 aa; SEQ ID NO: 43
      1 qvqlqqsgae lakpgasvkm scrasgytft dywmhwvkqr pgqglewigf
      51 inpstvytey ipkfkdkatl tadkssstay mqlssltsed savyycarsd
      101 ggwyfdvwga gttvtvss
Protein 499 RAGE-1
   VL 499: variable region for light chain; entire molecule length: 107 aa; SEQ ID NO: 21
      1 divmtqshkf mstsvgdrvs itckasqdvs tavawyqqkp gqspklliys
      51 asyrytgvpd rftgsgsgtd ftftissvqa edlavyycqq hyntprtfgg
      101 gtkleik
   VH 499: variable region for heavy chain; entire molecule length: 113 aa; SEQ ID NO: 44
      1 evqlqqsgtv larpgasvkm sckasgytft sywmhwvkqr pgqglewiga
      51 iypgdsdtyy nqkfkgkakl tavtststay melssltned savyyctrnw
      101 dywgqgttlt vss
Protein 501-4 RAGE-1
   VL 501-4: variable region for light chain; entire molecule length: 109 aa; SEQ ID NO: 22
      1 qavvtqesal ttspgetvtl tcrsstgavt tsnyanwvqe kpdhlftgli
      51 ggtnnrapdv parfsgslig dkaaltitga qtedeaiyfc alwysnhwvf
      101 gggtkltvl
   VH 501-4: variable region for heavy chain; entire molecule length: 120 aa; SEQ ID NO: 45
      1 evmlvdsggg lvkpggslkl scaasgftfr syamswvrqt pekrlewvat
      51 issggsytyy pdsvrgrftt srdngkntly lqmsslrsed tamyycarhg
      101 gnysawftyw gqgtlvtvsa
Protein 529 RAGE-1
   VL 529: variable region for light chain; entire molecule length: 109 aa; SEQ ID NO: 23
      1 qavvtqesal ttspgetvtl tcrsstgavt tsnyanwvqe kpdhlftgli
      51 ggtnnrspgv parfsgslig dkaaltitga qtedeaiyfc alwyszahlvf
      101 gggtkltvl
   VH 529: variable region for heavy chain; entire molecule length: 119 aa; SEQ ID NO: 46
      1 hseiqlqqtg pelvkpgasv kisckasgys ftdyimlwvk qshgkslewi
      51 gninpyygst fynlkfkgka tltvdkssst aymqlnslts edsavyycar
      101 sdywyfdvwg agttvtvss

### EXAMPLE 3: Characterization of Selected mAbs in Competition ELISA with S100A12 and S100A6

For the S100A12 Competition ELISA Assay wells have been coated with S100A12 at 0.5 µg/well. Thereafter, RAGE-Fc at 10 µg/mL and competing mAb at 10 µg/mL were pre-incubated at room temperature for 30 min. The mixture was transferred into S100A12-coated plates and incubated under agitation at room temperature for 2 hours. Bound RAGE-Fc was detected with anti-hIgG Fc-specific-POD using TMB (3, 3', 5, 5'-Tetramethylbenzidine) at 450 nm. None of the 27 antibody tested showed antagonist activity on RAGE-Fc / S100A12 interaction.

For the S100A6 Competition ELISA Assay wells have been coated with S100A6 at 0.5 µg/well. Thereafter, RAGE-Fc at 10 µg/mL and competing mAb at 10 µg/mL have been pre-incubated at room temperature for 30 min. The mixture was transferred into S100A6 -coated plates and incubated under agitation at room temperature for 2 hours. Bound RAGE-Fc was detected with anti-hIgG Fc-specific-POD using TMB (3, 3', 5, 5'-Tetramethylbenzidine) at 450 nm. It could be shown that 7 of 27 antibodies were able to disrupt RAGE-Fc/S100A6 interaction (15-35% inhibition).

For 5 mAbs (showing an inhibitory effect >20%; LP08103; LP08104; LP08105; LP08108 and LP08122D) dose-dependency of inhibition of RAGE-S100A6 binding (IC50) was tested. For this, coating with S100A6 was carried out as detailed above. RAGE-Fc at 10µg/mL was incubetd with competing mAb (2-fold serial dilutions starting at 100 µg/ml at room temperature for 2 hours. Binding was detected by by anti IgG Fc-specific antibody peroxydase conjugate using TMB1 component HRP at 450 nm.

The following IC50 values were obtained for the antibodies tested (see also Fig. 4):
56 RAGE-1 (LP08103): IC50 =1.16 [0.47; 2.80] µg/mL (7.73nM)
240 RAGE-1 (LP08108): IC50 = 6.66 [4.33; 10.20] µg/mL (44.4nM)
166 RAGE-1 (LP08122): IC50 = 8.33 [6.20; 11.24] µg/mL (55.5nM)
158 RAGE-1 (LP08105): IC50 = 6.79 [5.01; 9.21] µg/mL (45.3nM)
Control XT-M4 (LP08130): IC50 = 5.20 [3.18; 8.53] µg/mL (34.6nM)
No dose-effect obtained with 152 RAGE-1 (LP08104)

In summary, 4 mAbs disrupt hRAGE-S100A6 interaction with a maximum inhibitory effect of 50%.

### EXAMPLE 4: Characterization of mAbs identified in Competition Assay

The competition assays have been performed according to operating procedures that are well known to a person skilled in the art. Such operating procedures are comprised within the handbooks as listed in example 1. The results of competition asssays are compiled by table 1.

**Table 1: Characteristics of mAbs identified in Competition Assay**

| | RAGE-S100B Interaction (IC50) | RAGE-HMGB1 Interaction (IC50) | RAGE-S100A6 Interaction (IC50) | RAGE-S100A12 Interaction | V domain | V-C1 domains | C2 domain | Human/Cyno /rat/mouse cross reactivity | Human/Cyno/rat cross reactivity |
|---|---|---|---|---|---|---|---|---|---|
| 158 RAGE-1 (LP08105) | + 0.7 nM | + 1.4 nM | + 45.3nM | x | + | + | x | x | x |
| 166 RAGE-1 (LP08122) | + 0.8 | x | + 55.5nM | x | x | + | x | x | x |
| 240 RAGE-1 (LP08108) | + 0.8 nM | + 2.1 nM | + 44.4nM | x | + | + | x | x | x |
| 253 RAGE-1 (LP08127) | + 0.7 nM | x | x | x | x | + | x | x | + |
| 326 RAGE-1 (LP08137) | + 0.6 nM | x | x | x | x | + | x | x | x |
| 56 RAGE-1 (LP08103) | x | x | + 7.7nM | x | x | x | + | + | + |
| 152 RAGE-1 (LP08104) | x | x | + | x | + | + | x | x | x |
| 164 RAGE-1 (LP08106) | x | x | + | x | x | x | + | x | + |
| 173 RAGE-1 (LP08107) | x | x | + | x | + | + | x | x | x |
| XTM4 (LP08130) | + 1.37 nM | + 1.79 nM | + 34.6nM | x | + | + | x | | |

Competition ELISA assay showed that
5 mAbs strongly block hRAGE-S100B interaction, namely
158 RAGE-1 (LP08105) with a IC50 = 0.109 [0.071; 0.166] µg/mL (0.72 nM)
240 RAGE-1 (LP08108) with a IC50 = 0.123 [0.073; 0.204] µg/mL (0.82 nM)
166 RAGE-1 (LP08122) with a IC50 = 0.128 [0.093; 0.176] µg/mL (0.85 nM)
253 RAGE-1 (LP08127) with a IC50 = 0.108 [0.082; 0.131] µg/mL (0.72 nM)
326 RAGE-1 (LP08137) with a IC50 = 0.097 [0.064; 0.148] µg/mL (0.64 nM)
XT-M4(LP08130) with a IC50 = 0.208 [0.117; 0.368] µg/mL (1.37 nM)
- 2 mAbs block hRAGE-HMGB1 interaction namely
158 RAGE-1 (LP08105) with a IC50 = 0.290 [0.189; 0.447] µg/mL (1.39 nM)
240 RAGE-1 (LP08108) with a IC50 = 0.310 [0.270; 0.463] µg/mL (2.06 nM)
XT-M4 (LP08130) with a IC50 = 0.272 [0.168 ; 0,439] µg/mL (1.79 nM)
4 mAbs disrupt hRAGE-S100A6 interaction, namely
56 RAGE-1 (LP08103): IC50 = 1.16 [0.47; 2.80] µg/mL (7.73 nM)
240 RAGE-1 (LP08108) with a IC50 = 6.66 [4.33; 10.20] µg/mL (44.4 nM)
166 RAGE-1 (LP08122) with a IC50 = 8.33 [6.20; 11.20] µg/mL (55.5 nM)
158 RAGE-1 (LP08105) with a IC50 = 6.79 [5.01; 9.21] µg/mL (45.3 nM)
XT-M4 (LP08130) with a IC50 = 5.20 [3.18; 8.53] µg/mL (34.6 nM)

### EXAMPLE 5: Characterization of mAbs in Biacore Assay

The assay is based on a capture assay using immobilized anti-mouse Fc IgG on CM5 chip. For immobilization of anti-Fc antibody HBS-EP was used as running buffer. Standard amine coupling to a CM5 chip was carried out at a contact time of 11 min and a flow rate of 10 µl/min. 10mM Sodium Acetate pH 5.0 was used as immobilization buffer. The protein concentration (anti-mouse Fc, Biacore BR-1008-38) was 100 µg/mL.

For binding analysis SN anti-RAGE mAb diluted in running buffer at 1/10 (flow rate 5µl/min) and sRAGE (V-C1-C2) lot LP08062 diluted in running buffer at 15 nM (flow rate 50µl/min) was used as analyte and ligand, respectively (running buffer: HBS-P + BSA 12mg/mL + CM Dextran 12mg/mL and Regeneration solution: 10mM Glycine pH 1.7). Langmuir 1:1 analysis was used as fitting model. Results are summarized in Table 2.

**Table 2: Human RAGE binding to immobilized anti-hRAGE mAbs**

| | BIAcore | | |
|---|---|---|---|
| | kon | koff | K_{D} |
| 56 RAGE-1 | 2.89E+06 | 5.39E-03 | **1.87E-09** |
| 95 RAGE-1 | 2.66E+06 | 1.23E-03 | **4.62E-10** |
| 130 RAGE-1 | 5.76E+06 | 1.36E-03 | **2.36E-10** |
| 140 RAGE-1 | 5.19E+06 | 1.57E-03 | **3.03E-10** |
| 152 RAGE-1 | 3.86E+06 | 2.65E-03 | **6.87E-10** |
| 158 RAGE-1 | 4.02E+06 | 2.53E-03 | **6.29E-10** |
| 164 RAGE-1 | 1.80E+06 | 2.03E-03 | **1.13E-09** |
| 166 RAGE-1 | 3.24E+06 | 7.58E-04 | **2.34E-10** |
| 173 RAGE-1 | 3.35E+06 | 2.27E-03 | **6.78E-10** |
| 183 RAGE-1 | 1.58E+08 | 2.73E-03 | **1.73E-09** |
| 184 RAGE-1 | 2.74E+06 | 1.63E-03 | **5.95E-10** |
| 210 RAGE-1 | 3.88E+06 | 1.07E-03 | **2.76E-10** |
| 240 RAGE-1 | 3.47E+06 | 7.69E-04 | **2.22E-10** |
| 250 RAGE-1 | 3.58E+06 | 1.99E-03 | **5.56E-10** |
| 253 RAGE-1 | 3.89E+06 | 1.99E-03 | **5.12E-10** |
| 259 RAGE-1 | 4.34E+06 | 2.24E-03 | **5.16E-10** |
| 283 RAGE-1 | 5.29E+06 | 1.96E-03 | **3.71E-10** |
| 298 RAGE-1 | 4.67E+06 | 2.42E-04 | **5.18E-11** |
| 316 RAGE-1 | 5.54E+06 | 1.77E-03 | **3.19E-10** |
| 326 RAGE-1 | 2.87E+06 | 1.71E-03 | **5.96E-10** |
| 347 RAGE-1 | 3.41E+06 | 2.27E-03 | **6.66E-10** |
| 400 RAGE-1 | 1.75E+06 | 3.14E-04 | **1.79E-10** |
| 499 RAGE-1 | 1.98E+06 | 1.57E-03 | **7.93E-10** |
| 501-4 RAGE-1 | 1.20E+06 | 1.03E-04 | **8.58E-11** |
| 513 RAGE-1 | 2.69E+06 | 1.58E-03 | **5.87E-10** |
| 529 RAGE-1 | 3.06E+06 | 1.69E-03 | **5.52E-10** |
| 544 RAGE-1 | 2.79E+06 | 1.58E-03 | **5.66E-10** |

## Claims

1. A polypeptide or polypeptide complex comprising at least the two amino acid sequences or functionally active variants thereof, wherein the least the two amino acid sequences are
- SEQ ID NO:1 and SEQ ID NO:24,
- SEQ ID NO:2 and SEQ ID NO:25,
- SEQ ID NO:3 and SEQ ID NO:26,
- SEQ ID NO:4 and SEQ ID NO:27,
- SEQ ID NO:5 and SEQ ID NO:28,
- SEQ ID NO:6 and SEQ ID NO:29,
- SEQ ID NO:7 and SEQ ID NO:30,
- SEQ ID NO:8 and SEQ ID NO:31,
- SEQ ID NO:9 and SEQ ID NO:32,
- SEQ ID NO:10 and SEQ ID NO:33,
- SEQ ID NO:11 and SEQ ID NO:34,
- SEQ ID NO:12 and SEQ ID NO:35,
- SEQ ID NO:13 and SEQ ID NO:36,
- SEQ ID NO:14 and SEQ ID NO:37,
- SEQ ID NO:15 and SEQ ID NO:38,
- SEQ ID NO:16 and SEQ ID NO:39,
- SEQ ID NO:17 and SEQ ID NO:40,
- SEQ ID NO:18 and SEQ ID NO:41,
- SEQ ID NO:19 and SEQ ID NO:42,
- SEQ ID NO:20 and SEQ ID NO:43,
- SEQ ID NO:21 and SEQ ID NO:44,
- SEQ ID NO:22 and SEQ ID NO:45, and/or
- SEQ ID NO:23 and SEQ ID NO:46,
wherein these sequences are arranged to allow for specific binding to the "receptor for advanced glycation endproducts" (RAGE).

2. The polypeptide or polypeptide complex of claim 1, wherein the functionally active variant of any of the sequences SEQ ID NO: 1 to 23 comprises the complementarity determining region L3 (CDR L3), preferably CDR L1, CDR L2 and CDR L3, of the respective sequence of SEQ ID NO: 1 to 23; and/or
wherein the functionally active variant of any of the sequences SEQ ID NO: 24 to 46 comprises the complementarity determining region H3 (CDR H3), preferably CDR H1, CDR H2 and CDR H3, of the respective sequence of SEQ ID NO: 24 to 46.

3. The polypeptide or polypeptide complex of claim 1 or 2,
i) wherein the amino acid sequence of SEQ ID NO:1, SEQ ID NO:2, SEQ ID NO:3, SEQ ID NO:4, SEQ ID NO:5, SEQ ID NO:6, SEQ ID NO:7, SEQ ID NO:8, SEQ ID NO:9, SEQ ID NO:10, SEQ ID NO:11, SEQ ID NO:12, SEQ ID NO:13, SEQ ID NO:14, SEQ ID NO:1 5, SEQ ID NO: 16, SEQ ID NO:17, SEQ ID NO:18, SEQ ID NO:19, SEQ ID NO:20, SEQ ID NO:21, SEQ ID NO:22 and/or SEQ ID NO:23 or a functionally active variant thereof is a variable domain of a light chain (VL); and/orii) wherein the amino acid sequence of SEQ ID NO:24, SEQ ID NO:25, SEQ ID NO:26, SEQ ID NO:27, SEQ ID NO:28, SEQ ID NO;29, SEQ ID NO:30, SEQ ID NO:31, SEQ ID NO:32, SEQ ID NO:33, SEQ ID NO:34, SEQ ID) NO:35, SEQ ID NO:36, SEQ ID NO:37, SEQ ID NO:38, SEQ ID NO:39, SEQ ID NO:40, SEQ ID NO:41, SEQ ID NO:42, SEQ ID NO:43, SEQ ID NO:44, SEQ ID NO:45 and/or SEQ ID NO:46 or a functionally active variant thereof is a variable domain of a heavy chain (VH).

4. The polypeptide or polypeptide complex of any of claims 1 to 3, wherein the polypeptide or polypeptide complex is an antibody,

5. The polypeptide or polypeptide complex of claim 4, wherein the antibody is a monoclonal antibody, a chimeric antibody, a humanized antibody, a Fab, a Fab', a F(ab')2, a Fv, a disulfide-linked Fv, a scFv, a (scFv)2, a bivalent antibody, a bispecific antibody, a multispecific antibody, a diabody, a triabody, a tetrabody and/or a minibody..

6. The polypeptide or polypeptide complex of any of claims 1 to 5, wherein the polypeptide or polypeptide complex comprises a heavy chain immunoglobulin constant domain selected from the group consisting of: a human IgM constant domain, a human IgG1 constant domain, a human IgG2 constant domain, a human IgG3 constant domain, domain, a human IgG4 constant domain, a human IgE constant domain, and a human IgA constant domain.

7. The polypeptide or polypeptide complex of any of claims 1 to 6, wherein the functionally active variant
a) is a functionally active fragment consisting of at least 60 %, preferably at least 70 %, more preferably at least 80 %, still more preferably at least 90 %, even more preferably at least 95 %, most preferably 99 % of an amino acid sequence of any of the SEQ ID NOS: 1 to 46;
b) is a functionally active variant having at least 60 %, preferably at least 70 %, more preferably at least 80 %, still more preferably at least 90 %, even more preferably at least 95 %, most preferably 99 % sequence identity to an amino acid sequence of any of the SEQ ID NOS: 1 to 46; or
c) consists of an amino acid sequence of any of the SEQ ID NOS: 1 to 46 and 1 to 50 additional amino acid residue(s), preferably 1 to 40, more preferably 1 to 30, even more preferably at most 1 to 25, still more preferably at most 1 to 10, most preferably 1, 2, 3, 4 or 5 additional amino acids residue(s).

8. The polypeptide or polypeptide complex of 7, wherein the functionally active variant is derived from the amino acid sequence of any of the SEQ ID NOS: 1 to 46 of any of the SEQ ID NOS: 1 to 46 by one or more conservative amino acid substitution.

9. One or more nucleic acid(s) coding for the polypeptide or polypeptide complex according to any of claims 1 to 8.

10. The nucleic(s) of claim 9, wherein the nucleic acid(s) is/are located in a vector.

11. A cell producing an antibody as defined in any of claims 4 to 8.

12. A binding molecule capable of binding to RAGE and comprising the polypeptide or polypeptide complex according to any of claims 1 to 8,

13. A composition for use as a medicament, the composition comprising at least one polypeptide according to any of claims 1 to 8 and/or at least one nucleic acid according to claim 9.

14. The composition of claim 13 for treating a RAGE-related disease or disorder, preferably selected from the group consisting of sepsis, septic shock, listeriosis, inflammatory disease, including rheumatoid and psoriatic arthritis and intestinal bowel disease, cancer, arthritis, Crohn's disease, chronic acute inflammatory disease, cardiovascular disease, erectile dysfunction, diabetes, complication of diabetes, vasculitis, nephropathy, retinopathy, neuropathy, amyloidoses, atherosclerosis, peripheral vascular disease, myocardial infarction, congestive heart failure, diabetic retinopathy, diabetic neuropathy, diabetic nephropathy and Alzheimer's disease, especially diabetes and/or an inflammatory disorder.

15. A method of diagnosing a RAGE-related disease or disorder as defined in claim 13, comprising the steps of:
(a) contacting a sample obtained from a subject with the polypeptide or polypeptide complex according to any of claims 1 to 7 or the binding molecule of claim 12; and
(b) detecting the amount of RAGE,
wherein an altered amount of RAGE receptor relative to a control is indicative of a RAGE-related disease or disorder.
